(19) Europäisches Patentamt
European Patent Office
Office européen des brevets

(11) EP 4 306 101 A1

(12) # EUROPEAN PATENT APPLICATION

(43) Date of publication:
**17.01.2024 Bulletin 2024/03**

(21) Application number: **22382671.0**

(22) Date of filing: **14.07.2022**

(51) International Patent Classification (IPC):
***A61K 8/49*** *(2006.01)*

(52) Cooperative Patent Classification (CPC):
(C-Sets available)
**A61K 8/4973; A61K 8/9711; A61K 9/107; A61K 31/22; A61K 47/14; A61K 47/44; A61K 47/46; A61P 29/00; A61Q 19/005;**
A61K 2800/92 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Gat Therapeutics S.L.**
**08028 Barcelona (ES)**

(72) Inventors:
- **GARCÍA-DELGADO BANCHS, Noemí**
**E-08950 Esplugues del Llobregat, Barcelona (ES)**
- **RUIZ CÁNOVAS, Eugènia**
**E-08401 Granollers, Barcelona (ES)**
- **MERCADÉ ROCA, Jaume**
**E-08028 Barcelona (ES)**

(74) Representative: **ABG Intellectual Property Law, S.L.**
**Avenida de Burgos, 16D**
**Edificio Euromor**
**28036 Madrid (ES)**

(54) **COMPOSITIONS COMPRISING AMAROUCIAXANTHIN A ESTERS AND USES THEREOF**

(57) The present invention relates to a composition comprising the compound of formula (I) or a stereoisomer thereof, in particular amarouciaxanthin A acetate as well as their medical and cosmetic uses.

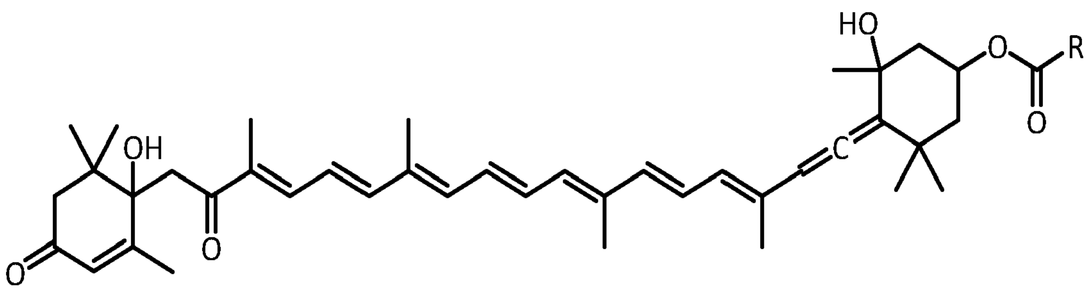

(I)

EP 4 306 101 A1

(52) Cooperative Patent Classification (CPC): (Cont.)

C-Sets
**A61Q 19/005, A61Q 19/08**

## Description

### FIELD OF THE INVENTION

The invention relates to a composition comprising amarouciaxanthin A esters and their medical and cosmetic uses.

### BACKGROUND OF THE INVENTION

Amarouciaxanthin A (ACX) is a naturally occurring xanthophyll which was first isolated from *Amaroucium pliciferum* (J Nat Prod, 1985, 48, 606-613). It is one of the main metabolites of fucoxanthin in mice after oral administration and the postulated metabolism is deacetylation of fucoxanthin (FCX) into fucoxanthinol followed by rearrangement in the liver to ACX (Drug. Metab. Dispos., 2004, 32, 205-211).

It is well known in the literature the wide range of applications of fucoxanthin (FCX) such as an antioxidant, antiobesity, antidiabetic, anticarcinogenic among others (Int J Mol Sci, 2013, 14, 13763-13781, Biochim Biophys Acta Mol Cell Biol Lipids, 2020, 1865, 158618). On the other hand, only recently ACX has been reported to significantly inhibit hepatic oxidative stress, inflammation, and fibrosis in rodent models (Biochem Biophys Res Commun, 2020, 528, 305-310) which makes this compound a very promising candidate.

FCX is widely distributed in brown seaweeds, diatoms and dinoflagellates and it is commercially available from natural extracts. Unfortunately there are no direct sources to obtain ACX or its derivatives since it is present in nature in only small amounts. It has been previously obtained either from natural extracts after a tedious purification process or by a very long chemical synthesis with low total yield and the risk of not fully controlling its chiral centers (Org Lett, 2013, 15, 5310-5313).

Therefore, there is a need of ACX prodrugs which can be synthesized in good yields, with no isomerization of the chiral centers, and which directly transform into ACX after administration, without intermediate metabolites as occurs when administering FCX.

Also, there is a need for suitable vehicles for effective solubilization and administration of ACX prodrugs that ensure their absorption and bioavailability.

### BRIEF DESCRIPTION OF THE INVENTION

Thus, in the first aspect, the invention relates to a composition comprising a compound of formula (I)

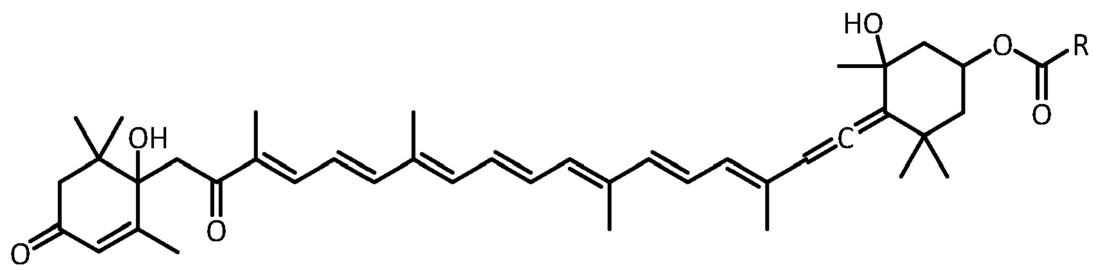

(I)

wherein R is selected from the group consisting of linear or branched $C_1$-$C_4$ alkyl, or a stereoisomer thereof and a vehicle, wherein the vehicle is selected from an oil or an emulsion.

In a second aspect, the invention relates to a foodstuff, nutritional supplement, cosmeceutical, cosmetic composition or nutraceutical comprising the composition of the invention.

In a third aspect, the invention relates to a non-therapeutic method for improving skin health comprising the step of administering to a patient in need of improved skin health the composition of the first aspect of the invention or the foodstuff, nutritional supplement, cosmeceutical, cosmetic composition of nutraceutical of the second aspect of the invention.

The fourth aspect relates to the composition of the invention for use in medicine or to a pharmaceutical composition comprising the composition of the invention.

The fifth aspect relates to the composition of the invention for use in the treatment and/or prevention of a disease selected form the group consisting of cancer, a cardiovascular disease, an intestinal flora imbalance, an inflammatory disease, an autoimmune disease, fibrosis, a bacterial infection, a neurological disease, a hyperuricemic-related disease,

a senescence-related disorder, a lipid related disease, glucocorticoid induced muscle atrophy, a bone-related disease, an ocular disease, an angiogenic related disease, psoriasis, eczema, atopic dermatitis, and a thyroid-related disorder.

The sixth aspect relates to the composition of the invention for use in the treatment and/or prevention of a disease mediated by glucocorticoid receptor activity, a senescence-associated disease or disorder, or as adjuvant in the treatment of cancer with chemotherapy, preferably taxane-comprising chemotherapy.

In a final aspect, the invention relates to a cosmetic method for preventing and/or decreasing cutaneous senescence and/or for ameliorating the cosmetic adverse effects of aging comprising administering the composition according to the first aspect invention or the foodstuff, nutritional supplement, cosmeceutical, cosmetic composition or nutraceutical of the second aspect of the invention to a subject in need thereof.

DESCRIPTION OF THE FIGURES

Figure 1 shows the plasma concentration of amarouciaxanthin A after oral delivery of amarouciaxanthin A acetate or fucoxanthin.

**DETAILED DESCRIPTION OF THE INVENTION**

Composition of the invention

The authors of the present invention have observed that formulations of amarouciaxanthin A acetate in an oil or in an emulsion unexpectedly result in improved adsorption and improved plasma levels of amarouciaxanthin when compared with those obtained when comparable formulations of a fucoxanthin are administered. Moreover, the authors of the present invention have also observed that amarouciaxanthin A acetate shows improved solubility in certain lipophilic solvents when compared with fucoxanthin.

Accordingly, in a first aspect, the invention relates to a composition comprising a compound of formula (I)

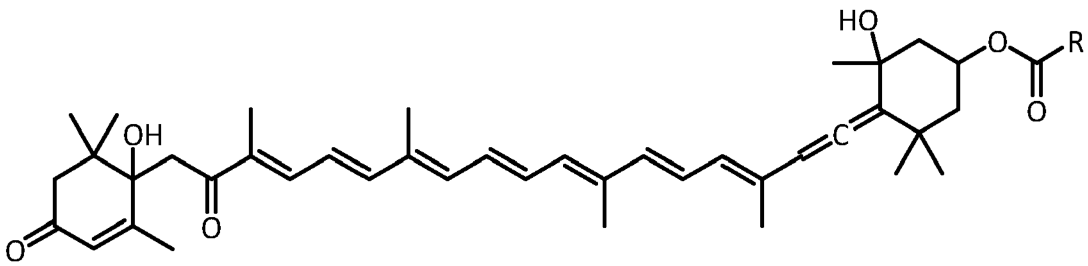

(I)

wherein R is selected from the group consisting of linear or branched $C_1$-$C_4$ alkyl, or a stereoisomer thereof and a vehicle, wherein the vehicle is selected from an oil or an emulsion.

The term "alkyl" refers to a straight or branched hydrocarbon chain radical consisting of carbon and hydrogen atoms, containing no unsaturation, having one to four carbon atoms, and which is attached to the rest of the molecule by a single bond, e. g., methyl, ethyl, n-propyl, iso-propyl, n-butyl, tert-butyl, iso-butyl, and sec-butyl. Preferably, the alkyl is methyl.

The term "stereoisomer" makes reference to compounds made up of the same atoms bonded by the same sequence of bonds but having different three-dimensional structures which are not interchangeable.

In a preferred embodiment, the compound of formula (I) is, more specifically, the compound of formula (Ia)

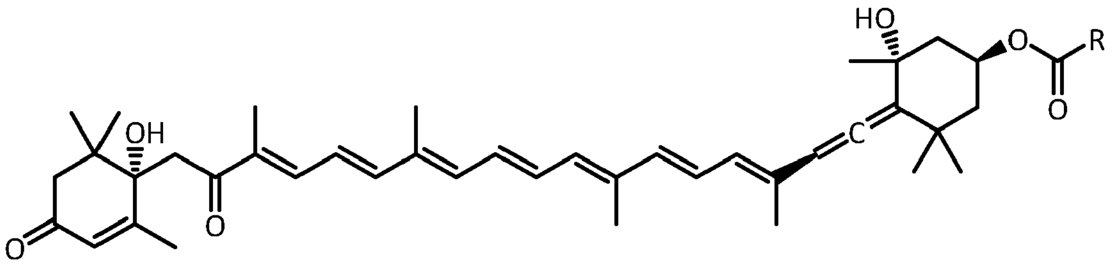

(Ia)

wherein R is as previously defined.

In a preferred embodiment, in the compound of the formula (I), R is methyl.

In a more preferred embodiment, in the compound of formula (Ia), R is methyl, i.e. amarouciaxanthin A acetate.

Methods for synthesizing the compound of formula (i) or formula (ia) are disclosed in the examples of the present application and are also described below.

The process for preparing a compound of formula (I) may comprise:

a) reacting a compound of formula (II) or (IIa)

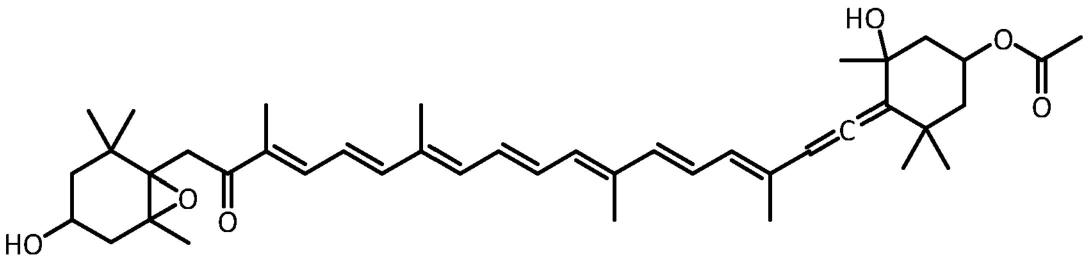

(II)

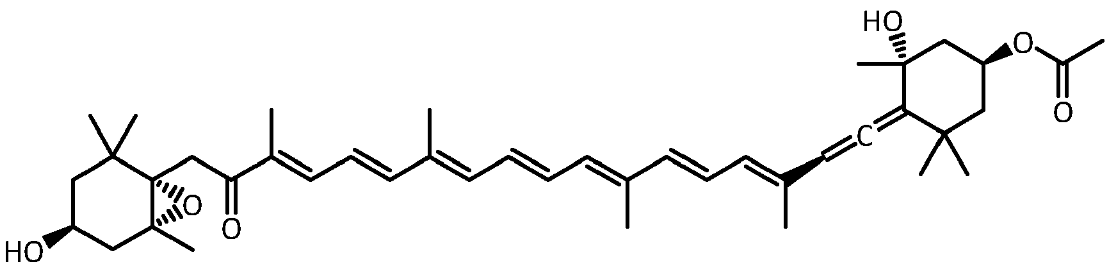

(IIa)

with an oxidant, preferably an oxidant selected from the group consisting of tetra-n-propylammonium perruthenate (TPAP) and 2-iodoxybenzoic acid (IBX) and optionally also in the presence of a co-oxidant, preferably *N*-methylmorpholine N-oxide (NMO), to give a compound of formula (III) or (IIIa)

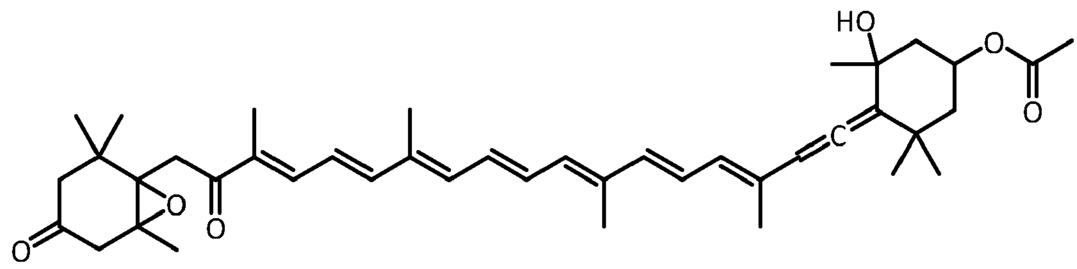

(III)

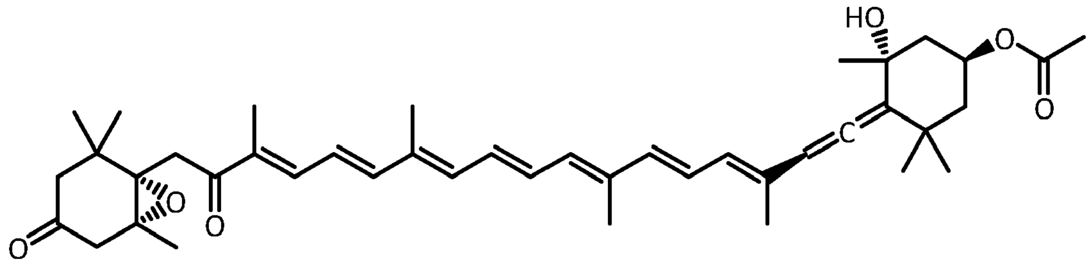

(IIIa)

b) treating the compound of formula (III) or (IIIa) with $SiO_2$ in the presence of a base, preferably a base selected from the group consisting of *N*-methylmorpholine and triethylamine, to give a compound of formula (I) or (Ia) wherein R is methyl, and

c) wherein if the compound of formula (I) or (Ia) R is a linear or branched $C_2$-$C_4$ alkyl, the process further comprises:

c1) converting the compound of formula (I) or (Ia) wherein R is methyl to a compound of formula (IV) or (IVa)

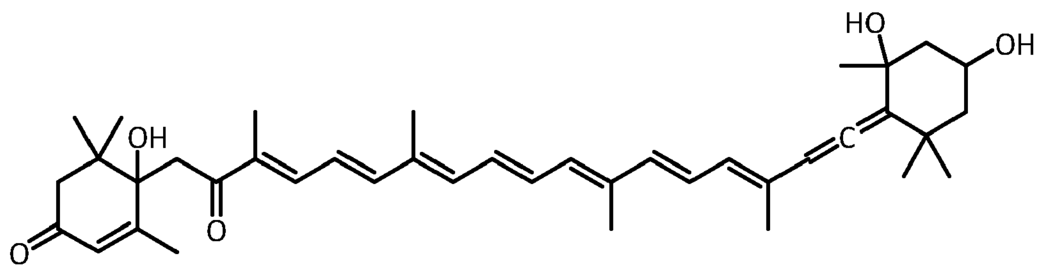

(IV)

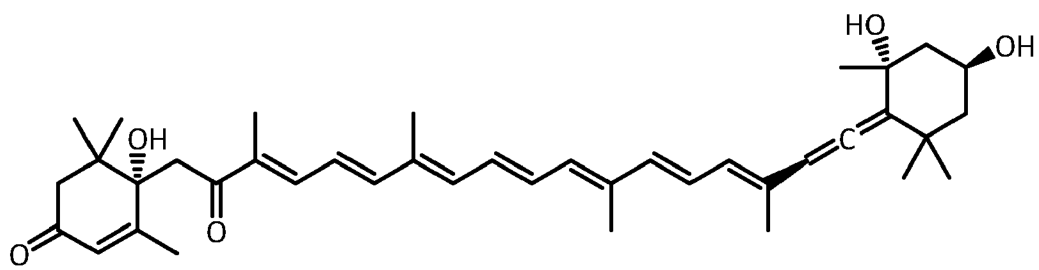

(IVa)

by treatment with an esterase, preferably a lipase, and

c2) esterification of the compound of formula (IV) or (IVa) with a carboxylic acid of formula (V)

R-COOH (V)

wherein R is a linear or branched $C_2$-$C_4$ alkyl,
in the presence of a coupling agent, preferably a carbodiimide, more preferably a carbodiimide selected from the group consisting of dicyclohexylcarbodiimide (DDC), diisopropylcarbodiimide (DIC) and ethyl-(*N'*,*N'*-dimethylamino)propylcarbodiimide (EDC) or a salt thereof, and in the presence of 4-(dimethylamino)pyridine (DMAP), to give a compound of formula (I) or (Ia)

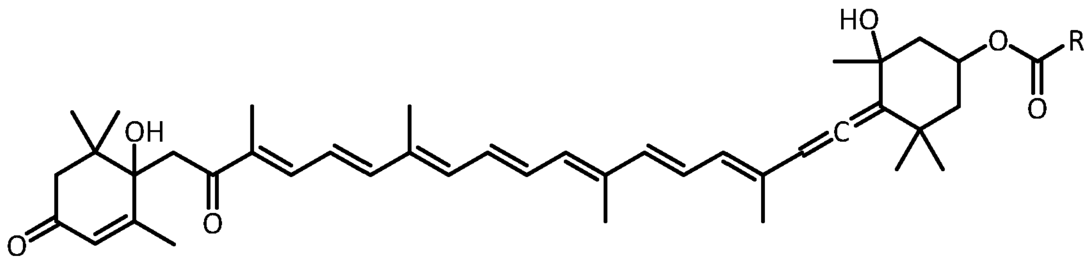

(I)

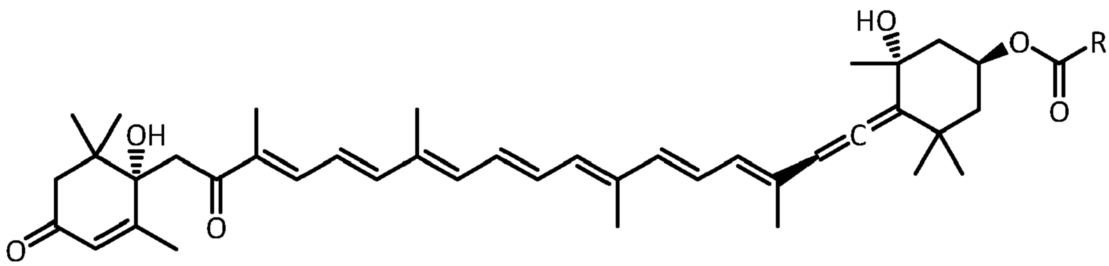

(Ia)

wherein R is selected from the group consisting of linear or branched $C_2$-$C_4$ alkyl.

When step a) is carried out with a compound of formula (II), a compound of formula (III) is obtained in this step, a compound of formula (I) wherein R is methyl is obtained in step b), a compound of formula (IV) is obtained in step c1) and a compound of formula (I) wherein R is $C_2$-$C_4$ alkyl is obtained in step c2).

When step a) is carried out with a compound of formula (IIa), a compound of formula (IIIa) is obtained in this step, a compound of formula (Ia) wherein R is methyl is obtained in step b), a compound of formula (IVa) is obtained in step c1) and a compound of formula (Ia) wherein R is $C_2$-$C_4$ alkyl is obtained in step c2).

The oxidant of step a) may be any suitable oxidant capable of providing the compound of formula (III) or (IIIa). Examples of oxidants are tetra-n-propylammonium perruthenate (TPAP) and 2-iodoxybenzoic acid (IBX), preferably TPAP.

Co-oxidants may also be used in step a). Examples and suitable co-oxidants are N-methylmorpholine *N*-oxide (NMO).

Preferably, step a) is carried out using TPAP as oxidant and NMO as co-oxidant.

The reaction of step a) is preferably carried out in an organic solvent such as dichloromethane, acetonitrile, acetone, methyl ethyl ketone, ethyl acetate, tetrahydrofuran or mixtures thereof, preferably dichloromethane.

The reaction of step a) is preferably carried out at a temperature in the range of 15°C to 35°C.

The amount of oxidant used in step a) is preferably from 0.01 to 0.5 mol with respect to each mol of the compound of formula (II) or (IIa), more preferably from 0.05 to 0.2 mol.

When present, the amount of co-oxidant used in step a) is preferably from 1.5 to 5 mol with respect to each mol of the compound of formula (II) or (IIa), more preferably from 1.5 to 2.5 mol.

Step a) is preferably carried out with the removal of water. For example molecular sieves of 4Å may be used in step a).

Step a) may also be carried out without the removal of water.

Preferably, step a) is carried out for 0.5 to 5 h.

Step a) provides a compound of formula (III) or (IIIa). When step a) is carried out with a compound of formula (II), a compound of formula (III) is obtained in this step. When step a) is carried out with a compound of formula (IIa), a compound of formula (IIIa) is obtained in this step.

The next step in the process is step b) of treating the compound of formula (III) or (IIIa) with $SiO_2$ in the presence of a base, preferably a base selected from the group consisting of *N*-methylmorpholine and triethylamine, to give a compound of formula (I) or (Ia) wherein R is methyl.

The compound of formula (III) or (IIIa) is obtained in step a).

The amount of $SiO_2$ used in step b) is preferably from 10 to 50 mol with respect to each mol of the compound of formula (II) or (IIa).

Step b) is carried out in the presence of a base. Examples of suitable bases are N-methylmorpholine, trimethylamine, *N,N*-diisopropylethylamine and mixtures thereof, preferably N-methylmorpholine.

The amount of base used in step b) is preferably from 0.1 to 5 mol with respect to each mol of the compound of formula (II) or (IIa).

Step a) may also be carried out in the presence of NMO as co-oxidant, providing N-methylmorpholine as a subproduct, which is then used as the base in step b).

Steps a) and b) may be carried out one-pot.

The reaction of step b) is preferably carried out in an organic solvent such as dichloromethane, acetonitrile, acetone, methyl ethyl ketone, ethyl acetate, tetrahydrofuran or mixtures thereof, preferably dichloromethane.

The reaction of step b) is preferably carried out at a temperature in the range of 15 °C to 35 °C.

Preferably, step b) is carried out for 0.5 to 24 h.

Step b) provides a compound of formula (I) or (Ia) wherein R is methyl. When step b) is carried out with a compound of formula (III), a compound of formula (I) wherein R is methyl is obtained in this step. When step b) is carried out with a compound of formula (IIIa), a compound of formula (Ia) is obtained in this step.

If the target compound of the process of the invention is a compound of formula (I) or (Ia) wherein R is methyl, step c) is not performed.

If the target compound of the process of the invention is a compound of formula (I) or (Ia) wherein R is a linear or branched $C_2$-$C_4$ alkyl, step c) is performed. Step c) comprises steps c1) and c2).

Step c1) is converting the compound of formula (I) or (Ia) wherein R is methyl to a compound of formula (IV) or (IVa)

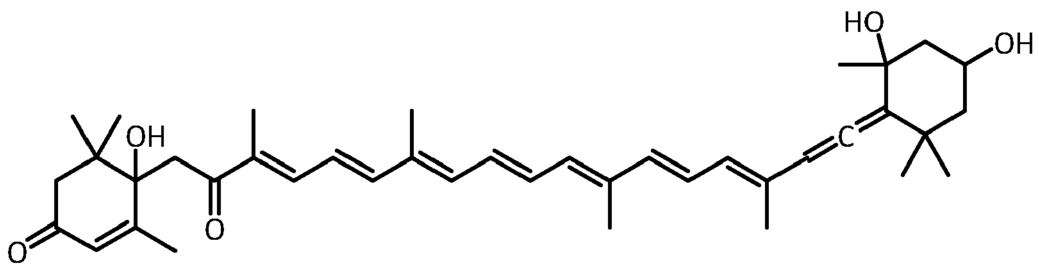

(IV)

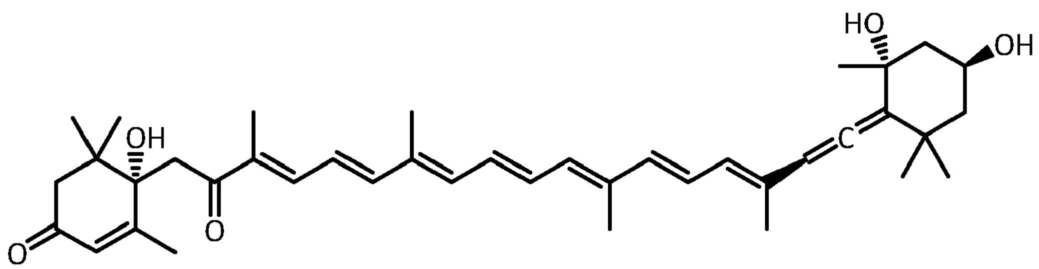

(IVa)

by treatment with an esterase, preferably a lipase.

The compound of formula (IV) or (IVa) is obtained in step b).

Any esterase capable of providing compound (IV) or (IVa) from a compound of formula (I) or (Ia), respectively,

may be used. Examples of esterases are lipase and cholesterol esterase, preferably a lipase.

Step c1) is preferably carried out in the presence of an emulsifier such as sodium taurocholate, sodium dodecyl sulfate or a hydrate thereof.

Preferably, step c1) is carried out in an aqueous solvent, preferably at a pH of 6.5 to 7.5, more preferably, step c1) is carried out in phosphate-buffered saline solution (PBS).

The reaction of step c1) is preferably carried out at a temperature in the range of 30 °C to 45 °C.

Preferably, step c1) is carried out for 2 to 24 h.

Step c1) provides a compound of formula (IV) or (IVa). When step c1) is carried out using a compound of formula (I), a compound of formula (IV) is obtained in this step. When step c1) is carried out using a compound of formula (Ia), a compound of formula (IVa) is obtained in this step.

The next step of the process of the invention is step c2) of esterification of the compound of formula (IV) or (IVa) with a carboxylic acid of formula (V)

R-COOH (V)

wherein R is a linear or branched $C_2$-$C_4$ alkyl,
in the presence of a coupling agent, preferably a carbodiimide, more preferably a carbodiimide selected from the group consisting of dicyclohexylcarbodiimide (DDC), diisopropylcarbodiimide (DIC) and ethyl-(*N'*,*N'*-dimethylamino)propylcarbodiimide (EDC) or a salt thereof, and in the presence of 4-(dimethylamino)pyridine (DMAP), to give a compound of formula (I) or (Ia)

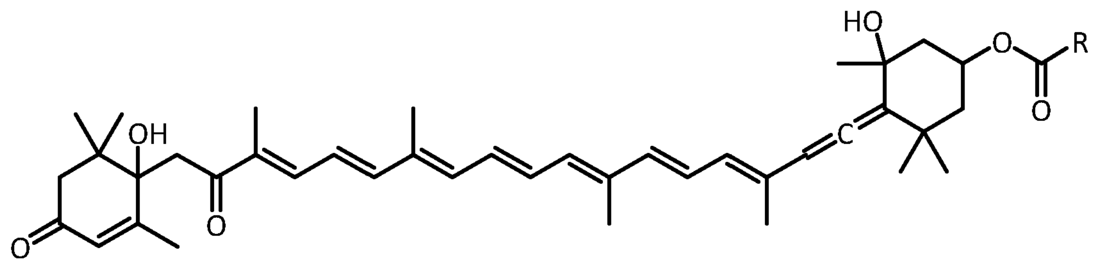

(I)

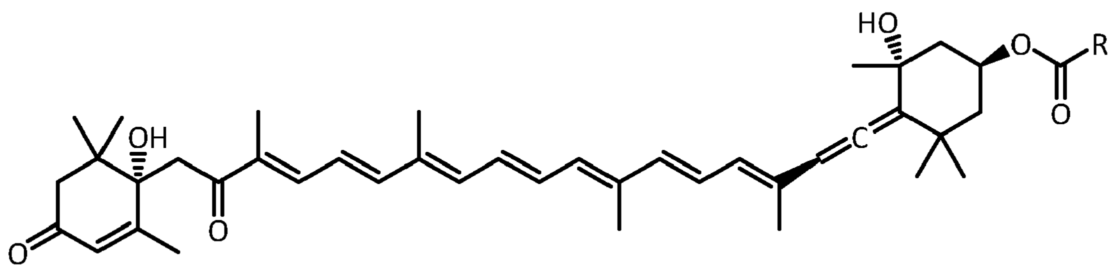

(Ia)

wherein R is selected from the group consisting of linear or branched $C_2$-$C_4$ alkyl.

The compound of formula (IV) or (IVa) is obtained in step c1).

The coupling agent used in step c2) may be any suitable coupling agent known in the art for esterification of a carboxylic acid with an alcohol. Examples of suitable coupling agents are carbodiimides, preferably dicyclohexylcarbodiimide (DDC), diisopropylcarbodiimide (DIC) and ethyl-(*N'*,*N'*-dimethylamino)propylcarbodiimide (EDC) or a salt thereof. The esterification reaction of step c2) is carried out in the presence of DMAP.

Preferably, from 1 to 2 mol of coupling agent with respect to each mol of carboxylic acid (V) is used in step c2).

Preferably from 0.01 to 0.5 mol of DMAP with respect to each mol of carboxylic acid of formula (V) is used in step c2).

Preferably, from 1 to 2 mol of compound of formula (IV) or (IVa) with respect to each mol of carboxylic acid of formula (V) is used in step c2).

Preferably, step c2) is carried out in an organic solvent, such as dichloromethane, N,N-dimethylformamide or

mixtures thereof.

The reaction of step c2) is preferably carried out at a temperature in the range of 0 °C to 30 °C.

Preferably, step c2) is carried out for 1 to 15 h.

Step c2) provides a compound of formula (I) or (Ia) wherein R is selected from the group consisting of linear or branched $C_2$-$C_4$ alkyl. When step c2) is carried out using a compound of formula (IV), a compound of formula (I) wherein R is selected from the group consisting of linear or branched $C_2$-$C_4$ alkyl is obtained in this step. When step c2) is carried out using a compound of formula (IVa), a compound of formula (Ia) wherein R is selected from the group consisting of linear or branched $C_2$-$C_4$ alkyl is obtained in this step.

In the process of the invention:

- the oxidant of step a) may be TPAP,
- step a) may be carried out in the presence of a co-oxidant,
- the co-oxidant in step a) may be NMO; and/or
- the base in step b) may be N-methylmorpholine.

In addition, step a) and/or b) of the process of the invention may be carried out in the presence of water, in particular with more than 0.05 mol of water with respect to each mol of compound of formula (II) or (IIa). This provides a mixture of a compound of formula (I) or (Ia) and a compound of formula (VI) or (VIa)

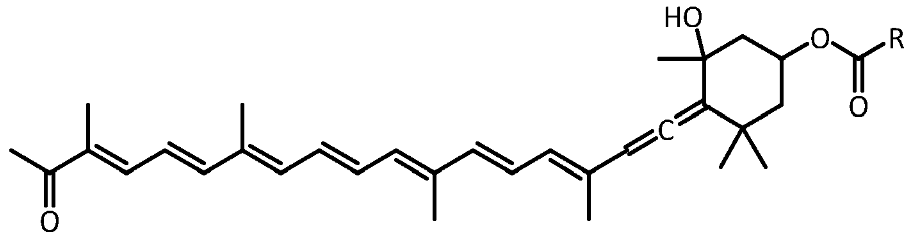

(VI)

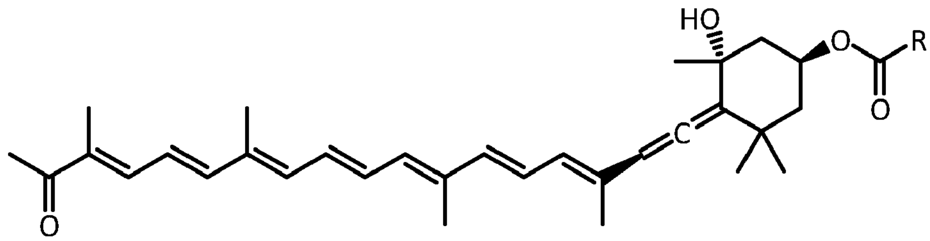

(VIa)

wherein R is selected from the group consisting of linear or branched $C_2$-$C_4$ alkyl, preferably methyl, preferably a compound of formula (Via), preferably a compound of formula (VIa) wherein R is methyl.

Alternatively, a compound of formula (VI) or (VIa) can be obtained by heating the compound of formula (I) or (Ia) at 60 to 90 °C in the presence of water. Step a) may be carried out in the presence of water or without the presence of water. To avoid the presence of water in step a) a water removing agent, such as 4Å molecular sieves, may be used. Step b) may be carried out in the presence of water or without the presence of water. To avoid the presence of water in step a) a water removing agent, such as 4Å molecular sieves, may be used. Alternatively, both steps a) and b) are carried out in the presence of water or without the presence of water. To avoid the presence of water in steps a) and b) a water removing agent, such as 4Å molecular sieves, may be used.

The term "presence of water" refers to an amount of water of 0.1% by volume to 100% by volume, preferably 100%. This amount of water is determined with respect to the solvents present in the reaction medium without taking into account the water removed by any water removal agent.

Thus, in an embodiment, the composition of the invention comprises a compound of formula (I) or (Ia), preferably a compound of formula (Ia), preferably wherein R is methyl, and a compound of formula (VI) or (VIa) as described above, preferably a compound of formula (Via), preferably wherein R is methyl.

In a particular embodiment, the amount of compound of formula (I) or (Ia) in the composition is from 10 to 99.9 wt% with respect to the total weight of the composition, preferably from 40 to 99 wt%.

In a particular embodiment, the amount of compound of formula (VI) or (VIa) in the composition is 1 to 60 wt% with respect to the total weight of the composition.

As used herein, the terms "oil", "lipid" or "fat", which are used indistinctly, will be defined to include any of a broad range of substances that are characteristically insoluble in water and extractable with an organic solvent. This broad class of compounds are well known to those of skill in the art, and as the term "lipid" is used herein, it is not limited to any particular structure. It includes organic compounds that has lipophilic or amphiphilic properties, including, but not limited to, fats, fatty oils, essential oils, waxes, steroids, sterols, phospholipids, glycolipids, sulpholipids, aminolipids, chromolipids (lipochromes), and fatty acids. Within the context of the present invention oil can be solid or liquid.

"Lipophilic" refers to those organic compounds that dissolve in fats, oils, lipids, and nonpolar solvents, such as organic solvents. Lipophilic compounds are sparingly soluble or insoluble in water. Thus, lipophilic compounds are hydrophobic. Amphipathic lipids, also referred to herein as "amphiphilic lipids", refer to a lipid molecule having both hydrophilic and hydrophobic characteristics. The hydrophobic group of an amphipathic lipid, as described in more detail immediately herein below, can be a long chain hydrocarbon group. The hydrophilic group of an amphipathic lipid can include a charged group, e.g., an anionic or a cationic group, or a polar, uncharged group. Amphipathic lipids can have multiple hydrophobic groups, multiple hydrophilic groups, and combinations thereof. Because of the presence of both a hydrophobic group and a hydrophilic group, amphipathic lipids can be soluble in water, and to some extent, in nonpolar organic solvents.

As used herein, "hydrophilic" is a physical property of a molecule that is capable of hydrogen bonding with a water ($H_2O$) molecule and is soluble in water and other polar solvents. The terms "hydrophilic" and "polar" can be used interchangeably. Hydrophilic characteristics derive from the presence of polar or charged groups, such as carbohydrates, phosphate, carboxylic, sulfate, amino, sulfhydryl, nitro, hydroxy and other like groups.

Conversely, the term "hydrophobic" is a physical property of a molecule that is repelled from a mass of water and can be referred to as "nonpolar", or "apolar", all of which are terms that can be used interchangeably with "hydrophobic." Hydrophobicity can be conferred by the inclusion of apolar groups that include, but are not limited to, long chain saturated and unsaturated aliphatic hydrocarbon groups and such groups substituted by one or more aromatic, cycloaliphatic or heterocyclic group(s).

As used herein, "vehicle" refers to a compound or composition that is capable of delivering a bioactive compound to a physiological site or cell.

The term oil refers either to a single oil or to a blended oil.

The term "blended oil" refers to an oil that is obtained by admixing, or blending any combination of, or individual, oil to obtain a desired composition. Thus, for example, types of oils from different microbes can be mixed together to obtain a desired PUFA composition. Alternatively, or additionally, the PUFA-containing oils disclosed herein can be blended with fish oil, vegetable oil or a mixture of both to obtain a desired composition.

Oil examples include compounds which contain long-chain aliphatic hydrocarbons and their derivatives. A lipid may be naturally occurring or synthetic (i.e., designed or produced by man). However, a lipid is usually a biological substance. Biological lipids are well known in the art, and include for example, neutral fats, phospholipids, phosphoglycerides, steroids, terpenes, lysolipids, glycosphingolipids, glycolipids, sulphatides, lipids with ether and ester-linked fatty acids and polymerizable lipids, and combinations thereof. Of course, compounds other than those specifically described herein that are understood by one of skill in the art as lipids are also encompassed by the compositions and methods of the present invention.

In a particular embodiment, the oil comprises neutral fats. Neutral fat refers to a low molecular alcohol and a fatty acid, also known as fatty acid esters (FAEs) or fatty acid alkyl ester (FAAEs). The fatty acid esters can be mono-, di- or triesters depending on the number of fatty acids. When the alcohol is glycerol, then the FAEs are called glycerides, and, likewise, depending on the number of fatty acids esterified with the glycerol molecule, the glycerides can be monoglycerides, diglycerides, or triglycerides respectively.

In a particular embodiment the lipid comprises a fatty acid ester or a fatty acid alkyl ester. In a preferred embodiment the lipid comprises mono-, di- and/or triesters. In another particular embodiment the oil comprises a blend of fatty acid esters. In another embodiment the oil comprises a blend of glycerides. In another embodiment, the oil comprises a blend of mono-, di- or triglycerides.

A fatty acid generally is a molecule comprising a carbon chain with an acidic moiety (e.g., carboxylic acid) at an end of the chain. The carbon chain of a fatty acid may be of any length, as long as the lipid molecule has a transition temperature of less than about 15° C. or less. For example, the fatty acid may include from about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, to about 30 or more carbon atoms, and any range derivable therein.

The transition temperature is the point at which the phospholipid membranes become more fluid, changing from a tightly ordered "gel" or "solid" phase to a liquid-crystal phase where the freedom of movement of individual molecules is higher (see discussion in New, 1990). This parameter can be measured for a single lipid, or for a composition

that includes more than one lipid.

The fatty acids of the FAEs, and more particularly, of the triglycerides can be saturated, monounsaturated and polyunsaturated, depending on the number of carbon-carbon double bond (C=C) in the aliphatic chains. A saturated fatty acid contains no carbon-carbon double bond in the aliphatic chain. Examples of saturated fatty acids include, e.g., palmitic and stearic acids. A monounsaturated fatty acid contains a single carbon-carbon double bond (C=C) in the aliphatic chain. Examples of monounsaturated fatty acids include, e.g., oleic and palmitoleic acids. A polyunsaturated fatty acid (also known as PUFAs) contains at least two carbon-carbon double bonds in the aliphatic chain.

Examples of polyunsaturated fatty acids include, e.g., linoleic acid (two C=C) and linolenic acid (three C=C). Further, the polyunsaturated fatty acids include omega-3 fatty acids and omega -6 fatty acids, depending on the location of the final C=C bond in the aliphatic chain. For example, linoleic is an omega-6 fatty acid, whereas linolenic is an omega-3 fatty acid.

Omega-3 fatty acids, also called Omega-3 oils, ω-3 fatty acids or n-3 fatty acids, are polyunsaturated fatty acids (PUFAs) characterized by the presence of a double bond, three atoms away from the terminal methyl group in their chemical structure. They are widely distributed in nature, being important constituents of animal lipid metabolism, and they play an important role in the human diet and in human physiology. The three types of omega-3 fatty acids involved in human physiology are α-linolenic acid (ALA), eicosapentaenoic acid (EPA) and docosahexaenoic acid (DHA). ALA can be found in plants, while DHA and EPA are found in algae and fish. Marine algae and phytoplankton are primary sources of omega-3 fatty acids. DHA and EPA accumulate in fish that eat these algae. Common sources of plant oils containing ALA include walnuts, edible seeds, and flaxseeds, while sources of EPA and DHA include fish and fish oils, as well as algae oil. The term "omega-3 fatty acids" includes natural and synthetic omega- 3 fatty acids, as well as pharmaceutically-acceptable esters, free acids, triglycerides, derivatives, conjugates (see, e.g., Zaloga et al., U.S. Patent Application Publication No. 2004/0254357, and Horrobin et al., U.S. Patent No. 6,245,81 1 , each hereby incorporated by reference), precursors, salts, and mixtures thereof. Examples of omega-3 fatty acid oils include, but are not limited to, omega-3 polyunsaturated, long- chain fatty acids such as eicosapentaenoic acid (EPA), docosahexaenoic acid (DHA), α-linolenic acid (ALA), heneicosapentaenoic acid (HPA), docosapentaenoic acid (DPA), eicosatetraenoic acid (ETA), eicosatrienoic acid (ETE), and octadecatetraenoic acid (i.e., stearidonic acid, STA); esters of omega-3 fatty acids with glycerol such as mono-, di- and triglycerides; and esters of the omega-3 fatty acids and a primary, secondary and/or tertiary alcohol, such as, for example, fatty acid methyl esters and fatty acid ethyl esters. The omega-3 fatty acids, esters, triglycerides, derivatives, conjugates, precursors, salts and/or mixtures thereof according to the present disclosure can be used in their pure form and/or as a component of an oil, for example, as marine oil (e.g., fish oil and purified fish oil concentrates), algae oils, microbial oils and plant-based oils.

In some embodiments of the present disclosure, at least one of the omega-3 fatty acids of the fatty acid oil mixture has a cis configuration. Examples include, but are not limited to, (all-Z)-9, 12, 15-octadecatrienoic acid (ALA), (all-Z)- 6,9,12, 15-octadecatetraenoic acid (STA), (all-Z)-1 1 , 14, 17-eicosatrienoic acid (ETE), (all-Z)-5,8, 1 1 ,14,17-eicosapentaenoic acid (EPA), (all-Z)-4,7, 10,13,16, 19- docosahexaenoic acid (DHA), (all-Z)-8, 1 1 , 14,17-eicosatetraenoic acid (ETA), (all-Z)- 7, 10, 13,16,19-docosapentaenoic acid (DPA), (all-Z)-6,9, 12,15, 19- heneicosapentaenoic acid (HPA); (all-Z)-5,8, 1 1 , 14-eicosatetraenoic acid, (all-Z)- 4,7, 10,13, 16-docosapentaenoic acid (osbond acid), (all-Z)-9, 12-octadecadienoic acid (linoleic acid), (all-Z)-5,8,1 1 , 14-eicosatetraenoic acid (AA), (all-Z)-6,9,12- octadecatrienoic acid (GLA); (Z)-9-octadecenoic acid (oleic acid), 13(Z)-docosenoic acid (erucic acid), (R-(Z))-12-hydroxy-9-octadecenoic acid (ricinoleic acid).

Omega-6 fatty acids (also referred to as ω-6 fatty acids or n-6 fatty acids) are a family of polyunsaturated fatty acids that have in common a final carbon-carbon double bond in the n-6 position, that is, the sixth bond, counting from the methyl end. Examples of omega-6 fatty acid include linoleic acid (LA), gamma-linolenic acid (GLA), calendic acid, eicosadienoic acid, dihomo-gamma-linolenic acid (DGLA), arachidonic acid (AA, ARA), docosadienoic acid, adrenic acid, osbond acid, tetracosatetraenoic acid, tetracosapentaenoic acid; esters of omega-6 fatty acids with glycerol such as mono-, di- and triglycerides; and esters of the omega-6 fatty acids and a primary, secondary and/or tertiary alcohol, such as, for example, fatty acid methyl esters and fatty acid ethyl esters. The omega-6 fatty acids, esters, triglycerides, derivatives, conjugates, precursors, salts and/or mixtures thereof according to the present disclosure can be used in their pure form and/or as a component of an oil.

In a particular embodiment, the oil is a vegetable oil, a microorganism oil, krill or a fish oil.

In another embodiment, the oil is a blended oil comprising any of a vegetable oil, microorganism oil and/or fish oil.

In another embodiment, the oil is a blend of fatty acids, the relative amounts of each can impact the overall characteristics of the oil, especially its ability to resist oxidation and to stabilize the compound suspended therein.

In certain embodiments, the oil is enriched in polyunsaturated or monounsaturated fatty acids. Within the context of the present invention, the term "enriched" is used to define an oil comprising more than 20% w/w, more than 25% w/w, 30% w/w, more than 35% w/w, more than 40% w/w, more than 45% w/w, more than 50% w/w, more than 55% w/w, more than 60% w/w or more than 70% w/w of polyunsaturated and/or monounsaturated fatty acids of the total weight of the oil.

In other embodiments, the oil can be a vegetable-based oil or oil blend.

In another embodiment, the oil can be a vegetable-based oil of a blend that comprises more than 25% w/w, 30% w/w, more than 35% w/w, more than 40% w/w, more than 45% w/w, more than 50% w/w, more than 55% w/w, more than 60% w/w or more that 70% w/w of polyunsaturated fatty acids, the polyunsaturated fatty acids including an omega-6 fatty acid and an omega-3 fatty acid.

"Vegetable oil" refers to any edible oil obtained from a plant. Typically plant oil is extracted from seed or grain of a plant. Vegetable oils are derived from some seeds, nuts, cereal grains, and fruits. Vegetable oils are composed of complex mixtures of triacylglycerols (TAGs; usually > 95%) with some minor amounts of diacylglycerols (usually < 5%). Other minor components are tocopherols/tocotrienols (up to 900 mg kg-1) and phytosterol esters/phytosterols (up to 1%). The vegetable oil may be characterized confidently with expert chromatographic analysis by determining its TAG composition, together with the fatty acid composition and the minor components

The term "triacylglycerols" ("TAGs") refers to neutral lipids composed of three fatty acyl residues esterified to a glycerol molecule. TAGs can contain long chain PUFAs and saturated fatty acids, as well as shorter chain saturated and unsaturated fatty acids.

"Microorganism oil (MO)" or "microbial oils", also called single cell oils (SCOs), consists of the intracellular storage lipids, triacylglycerols. It is similar to vegetable oil, another biologically produced oil. They are produced by oleaginous microorganisms, which is the term for those bacteria, molds, algae and yeast, which can accumulate 20% to 80% lipids of their biomass. The accumulation of lipids takes place by the end of the logarithmic phase and continues during the stationary phase until carbon source begins to reduce with nutrition limitation. Oleaginous microorganisms are also potential sources for such fatty acids.

The most important source is some species of yeast, that are able to convert food into triglycerides and accumulate the produced lipids when fed carbohydrates.

PUFAs produced by MOs through fermentation are a good and renewable source of PUFAs. Some fungal species, such as *Mortierella spp.*, including *M. elongate*, *M. isabellina*, and *M. alpina* (Lounds et al., 2007), are considered good sources of PUFAs. Some *Phythium* and *Phytophtora spp.* can also produce ω-3 PUFAs (O'Brien et al., 1993). Porphyridium cruentum, the red marine algae, produces significant amounts of EPA and arachidonic acid (AA). However, only a small number of Gram negative marine bacteria, such as *Flexibacter* and *Shewanella* sp. can produce PUFAs (Akimoto et al., 1990).

In a particular embodiment, the lipid vehicle is a fish oil or a microorganism oil which is enriched in DHA or EPA.

Fish oil is oil derived from the tissues of oily fish. Fish oils contain the omega-3 fatty acids eicosapentaenoic acid (EPA) and docosahexaenoic acid (DHA), precursors of certain eicosanoids that are known to reduce inflammation in the body and improve hypertriglyceridemia.

"Fish oil" refers to oil derived from the tissues of an oily fish. Examples of oily fish include, but are not limited to: menhaden, anchovy, herring, capelin, cod, and the like. Fish oil is a typical component of feed used in aquaculture.

"Menhaden" refers to forage fish of the genera Brevoortia and Ethmidium, two genera of marine fish in the family Clupeidae. Recent taxonomic work using DNA comparisons have organized the North American menhadens into large-scaled (Gulf and Atlantic menhaden) and small-scaled (Finescale and Yellowfin menhaden) designations (Anderson, J. D., Fishery Bulletin, 105(3):368-378).

"Anchovies" from which anchovy fish meal and anchovy fish oil are produced, are a family (Engraulidae) of small, common salt-water forage fish. There are about 140 species in 16 genera, found in the Atlantic, Indian, and Pacific Oceans.

The term "krill" refers to small crustaceans of the order *Euphausiacea*, and are found in all the world's oceans. "Krill oil" as used herein, refers here to any mixture of extracted lipids derived from any portion of a krill organism, it can include an oil directly obtained from krill, an oil which is derived from a krill source and which has been further modified/processed, and combinations thereof. Krill oil is a unique marine oil containing omega-3 or n-3 fatty acids (FAs), wherein the bioactive eicosapentaenoic acid (EPA) and docosahexaenoic acid (DHA) are present (i.e., comprising up to 35% w/w of the FA profile) mainly in phospholipids or PLs (up to 95% w/w), and containing up to 60% PLs and up to 45% triglycerides in the oil.

The krill oil contains phospholipids to which omega-3 fatty acids are attached. For example, the krill oil can contain omega-3 fatty acid- containing phospholipids in an amount of about 20 to about 60%, for example, from about 30 to about 50%, based on the weight of the krill oil. In an exemplary embodiment, the krill oil can contain omega-3 fatty acid- containing triglycerides in an amount of less than about 30%, for example, less than about 5%, based on the weight of the krill oil.

In an exemplary embodiment, a pre-determined amount of the omega-3 fatty acid-containing phospholipids contain omega-3 fatty acids attached to the first and second positions of the phospholipid, but not the third position of the phospholipid. That is, a predetermined amount of the omega-3 fatty acid-containing phospholipids can contain an omega-3 fatty acid in the second position (i.e., the middle position) of the phospholipid. For example, the omega-3 fatty acid-containing phospholipids containing omega-3 fatty acids attached to the first and second positions of the phospholipid, but not the third position of the phospholipid, can be present in an amount of about 70% to about 80%, for example, from about 80% to about 95%, based on the total weight of the omega-3 fatty acid- containing phospholipids.

In an exemplary embodiment, the marine crustacean or krill oil can be distinguishable from fish oils at least based on (1) the phospholipid contents, (2) the content of omega-3 fatty acid-containing phospholipids, (3) the content of omega-3 fatty acid-containing triglycerides, and/or (4) the content of the omega-3 fatty acid-containing phospholipids which contain omega-3 fatty acids attached to the first and second positions of the phospholipid, but not the third position of the phospholipid. For example, the marine crustacean or krill oil can contain a higher concentration of omega-3 fatty acid-containing phospholipids, a lower concentration of omega-3 fatty acid-containing triglycerides, and/or a higher content of the omega-3 fatty acid-containing phospholipids which contain omega-3 fatty acids attached to the first and second positions of the phospholipid, but not the third position of the phospholipid.

Docosahexaenoic acid (DHA) is an omega-3 fatty acid that is a primary structural component of the human brain, cerebral cortex, skin, and retina. In physiological literature, it is given the name 22:6(n-3). It can be synthesized from alpha-linolenic acid or obtained directly from maternal milk (breast milk), fatty fish, fish oil, or algae oil. DHA's structure is a carboxylic acid (-oic acid) with a 22-carbon chain (docosa- derives from the Ancient Greek for 22) and six (hexa-) cis double bonds (-en-); with the first double bond located at the third carbon from the omega end.[3] Its trivial name is cervonic acid (from the Latin word cerebrum for "brain"), its systematic name is all-cis-docosa-4,7,10,13,16,19-hexa-enoic acid, and its shorthand name is 22:6(n-3) in the nomenclature of fatty acids.

Eicosapentaenoic acid (EPA) is an omega-3 fatty acid. In physiological literature, it is given the name 20:5(n-3). It also has the trivial name timnodonic acid. In chemical structure, EPA is a carboxylic acid with a 20-carbon chain and five cis double bonds; the first double bond is located at the third carbon from the omega end. EPA is a polyunsaturated fatty acid (PUFA) that acts as a precursor for prostaglandin-3 (which inhibits platelet aggregation), thromboxane-3, and leukotriene-5 eicosanoids. EPA is both a precursor and the hydrolytic breakdown product of eicosapentaenoyl ethanolamide (EPEA: $C_{22}H_{35}NO_2$; 20:5,n-3).

Most of the docosahexaenoic acid in fish and multi-cellular organisms with access to cold-water oceanic foods originates from photosynthetic and heterotrophic microalgae, and becomes increasingly concentrated in organisms the further they are up the food chain. DHA is also commercially manufactured from microalgae: *Crypthecodinium cohnii* and another of the genus *Schizochytrium.* DHA manufactured using microalgae is vegetarian.

In some embodiments of the present disclosure, the weight ratio of EPA: DHA of the fatty acid oil ranges from about 1:10 to about 10:1, from about 1:8 to about 8:1, from about 1:6 to about 6:1 , from about 1:5 to about 5:1 , from about 1:4 to about 4:1 , from about 1:3 to about 3:1 , or from about 1:2 to 2:1. In at least one embodiment, the weight ratio of EPA:DHA of the fatty acid oil mixture ranges from about 1:2 to about 2:1. In at least one embodiment, the weight ratio of EPA:DHA of the fatty acid oil mixture ranges from about 1:1 to about 2:1. In at least one embodiment, the weight ratio of EPA:DHA of the fatty acid oil mixture ranges from about 1:2 to about 1:3.

The term "enriched in DHA or EPA" is used to refer to a percentage by weight of DHA or EPA in relation to the total weight of fatty acids of said oil which ranges between 30-100%, preferably between 40 and 90%, between 50 and 80%, and more preferably said percentage by weight of DHA or EPA is between 60 and 70%.

In a particular embodiment the vehicle is a vegetable oil which is selected from the group consisting of sunflower oil, coconut oil, canola oil, cottonseed oil, olive oil, palm oil, rapeseed oil, safflower oil, almond oil, beech nut oil, brazil nut oil, cashew oil, hazelnut oil, macadamia oil, mongongo nut oil, pecan oil, pine nut oil, pistachio oil, walnut oil, pumpkin seed oil, grapefruit seed oil, lemon oil, orange oil, acai oil, black seed oil, blackcurrant seed oil, borage seed oil, evening primrose oil, flaxseed oil, amaranth oil, apricot oil, apple seed oil, argan oil, avocado oil, babassu oil, ben oil, borneo tallow nut oil, cape chestnut oil, carob pod oil, cocklebur oil, cohune oil, coriander seed oil, date seed oil, dika oil, grape seed oil, kapok seed oil, kenaf seed oil, lallemantia oil, mafura oil, marula oil, mustard oil, nige seed oil, okra seed oil, papaya seed oil, perilla seed oil, persimmon seed oil, pequi oil, pili nut oil, pomegranate seed oil, poppy seed oil, pracaxi oil, prune kernel oil, quinoa oil, ramtil oil, rice bran oil, royle oil, sacha inchi oil, sapote oil, seje oil, taraminra oil, tea seed oil, thistle oil, tigernut oil, tomato seed oil, wheat germ oil, peanut oil, sesame oil, soybean oil, corn oil and mixtures thereof; preferably sunflower oil.

In a particular embodiment, the oil is sunflower oil.

Sunflower oil is the non-volatile oil pressed from the seeds of the sunflower (*Helianthus annuus*). Sunflower oil is commonly used in food as a frying oil, and in cosmetic formulations as an emollient. Sunflower oil is primarily composed of linoleic acid, a polyunsaturated fat, and oleic acid, a monounsaturated fat Sunflower oil is mainly a triglyceride. The British Pharmacopoeia lists the following profile: palmitic acid (saturated) 5%, stearic acid (saturated) 6%, oleic acid (monounsaturated omega-9) 30%, linoleic acid (polyunsaturated omega-6) 59%. Four types of sunflower oils with differing concentrations of fatty acids are produced through plant breeding and industrial processing: high-linoleic, high-oleic, mid-oleic, and high-stearic combined with high-oleic. High-linoleic: 69% linoleic acid; High-oleic: 82% oleic acid; Mid-oleic: 65% oleic acid; High-stearic with high-oleic: 18% stearic acid and 72% oleic acid. Within the context of the present invention. Any of the any of the four types of the sunflower oil or combinations thereof is contemplated as oil vehicle for the composition of the invention.

In a particular embodiment, the vehicle is a vegetable oil or a vegetable-based oil blend which comprises saturated fatty acids (SAFA), monounsaturated fatty acids (MUFA), and polyunsaturated fatty acids (PUFA).

In another embodiment, the vegetable oil or the vegetable-based oil blend comprises of about 5% w/w to 95% w/w, about 10% w/w to 80% w/w, about 10% w/w to 70% w/w, about 10% w/w to 60% w/w, about 10% w/w to 50% w/w, about 10% w/w to 40% w/w, about 10% w/w to 30% w/w, about 10% w/w to 20% w/w of MUFA in the total weight of the oil.

In another particular embodiment the ratio omega-6/omega-3 fatty in the vegetable oil or the vegetable-based oil blend is between 0.1 and 650, between 1 and 650, between 10 and 650, between 20 and 650, between 30 and 650, between 40 and 650, between 50 and 650, between 60 and 650, between 70 and 650, between 80 and 650, between 90 and 650, between 100 and 650, between 150 and 650, between 200 and 650, between 250 and 650, between 300 and 650, between 350 and 650 or between 400 and 650.

In another embodiment, the vegetable oil or the vegetable-based oil blend comprises of about 1% w/w to 80% w/w, about 20% w/w to 70% w/w, about 30% w/w to 65% w/w, about 4% w/w to 65% w/w or about 50% w/w to 65% w/w of omega-6 fatty acid in the total weight of the oil.

In another embodiment, the vegetable oil or the vegetable-based oil blend comprises of about 0.05% w/w to 70% w/w, about 0.05% w/w to 60% w/w, about 0.05% w/w to 50% w/w, about 0.05% w/w to 40% w/w, about 0.05% w/w to 30% w/w, about 0.05% w/w to 30% w/w, about 0.05% w/w to 20% w/w, about 0.1% w/w to 10% w/w or about 0.1% w/w to 1% w/w of omega-3 fatty acids.

Fatty acids are sometimes designated using numerical expressions in which the number of carbon atoms, the number of double bonds and the positions of double bonds are indicated in a simplified way by combining numbers and letters in the alphabet. For instance, a saturated fatty acid having 20 carbon atoms may be designated as C20:0, a monovalent unsaturated fatty acid having 18 carbon atoms designated as C18:1, eicosapentaenoic acid designated as C20:5 n-3, and so on. The symbol n- represents the position of a double bond as counted from the terminal methyl group in a fatty acid; for example, n-3 indicates that the bond between the third and fourth carbon atoms as counted from the terminal methyl group in a fatty acid is a double bond. This method of designation is well known to persons skilled in the art and fatty acids designated in accordance with this method can be readily identified by any person skilled in the art.

In another particular embodiment, the oil comprises highly unsaturated fatty acids or alkyl esters thereof. As used herein, the term highly unsaturated fatty acids means fatty acids having 18 or more carbon atoms and 3 or more double bonds. Highly unsaturated fatty acids can, for example, be fatty acids having 20 or more carbon atoms and 3 or more or even 4 or more double bonds, or fatty acids having 20 or more carbon atoms and 5 or more double bonds. Exemplary highly unsaturated fatty acids include linolenic acid (18:3 n-3), linolenic acid (18:3 n-6), dihomo-linolenic acid (20:3 n-6), arachidonic acid (20:4 n-6), eicosapentaenoic acid (C20:5 n-3), docosapentaenoic acid (22:5 n-6), and docosahexaenoic acid (22:6 n-3).

As used herein, a composition containing highly unsaturated fatty acids or alkyl esters thereof means either a fatty acid composition containing highly unsaturated fatty acids or a fatty acid alkyl ester composition containing alkyl esters of highly unsaturated fatty acids. Here, the fatty acid composition is a composition containing fatty acids as major constituents, and the fatty acid alkyl ester composition is a composition containing alkyl esters of fatty acids as major constituents.

In the present invention, the highly unsaturated fatty acid is not particularly limited if alkyl esters thereof are obtained as a main distillate upon enrichment by distillation. The highly unsaturated fatty acid may be eicosapentaenoic acid, docosahexaenoic acid, dihomo-linolenic acid, arachidonic acid, or combinations thereof. In preferred embodiments, the highly unsaturated fatty acid can be eicosapentaenoic acid, docosahexaenoic acid, or a combination thereof. In more preferred embodiments, the highly unsaturated fatty acid can be eicosapentaenoic acid.

In certain embodiments, the oil is enriched in polyunsaturated or monounsaturated fatty acids and/or alkyl esters. Within the context of the present invention, the term "enriched" is used to define an oil comprising more than 20% w/w, more than 25% w/w, 30% w/w, more than 35% w/w, more than 40% w/w, more than 45% w/w, more than 50% w/w, more than 55% w/w, more than 60% w/w of polyunsaturated and/or monounsaturated fatty acids and/or or alkyl esters of the total weight of the oil.

In a particular embodiment, the oil is enriched in polyunsaturated fatty acids and/or omega-3 fatty acids, being the term enriched as defined above.

Another class of lipids includes phospholipids. Thus, in another embodiment, the oil comprises phospholipids. A phospholipid generally comprises either glycerol or an sphingosine moiety, an ionic phosphate group to produce an amphipathic compound, and one or more fatty acids. Types of phospholipids include, for example, phosphoglycerides, wherein a phosphate group is linked to the first carbon of glycerol of a diglyceride, and sphingophospholipids (e.g., sphingomyelin), wherein a phosphate group is esterified to a sphingosine amino alcohol. For example, in some embodiments, the lipid molecule is dilaurylphosphatidylcholine, a phospholipid which includes two fully saturated fatty acid moieties.

A phospholipid may, of course, comprise further chemical groups, such as for example, an alcohol attached to the phosphate group. One of ordinary skill in the art would be familiar with the broad class of agents known as phospholipids, and the chemical groups which may be attached to phospholipids.

One of ordinary skill in the art would be familiar with the numerous other classes of lipids that are known to exist.

Lipids can be obtained from natural sources, commercial sources or chemically synthesized, as would be known to one of ordinary skill in the art. For example, phospholipids can be from natural sources, such as egg or soybean phosphatidylcholine, brain phosphatidic acid, brain or plant phosphatidylinositol, heart cardiolipin and plant or bacterial phosphatidylethanolamine. In another example, lipids suitable for use according to the present invention can be obtained from commercial sources.

Examples of further fatty acids, or fatty acid alkyl ester composition or mixtures thereof (fatty acid oil mixtures) encompassed by the present disclosure include, but are not limited to, the fatty acids defined in the European Pharmacopoeia Omega-3 Ethyl Esters 90 and purified marine oils, for example, the European Pharmacopoeia Omega-3 Acid Triglycerides, the European Pharmacopoeia Omega-3 acid Ethyl Esters 60, the European Pharmacopoeia Fish Oil Rich in Omega-3 Acids monograph, and/or for instance, the USP fish oil monograph.

Commercial examples of fatty acid oil mixtures comprising different fatty acids suitable for the present disclosure include, but are not limited to: Incromega™ omega-3 marine oil concentrates such as Incromega™ TG7010 SR, Incromega™ E7010 SR, Incromega™ TG6015, Incromega™ EPA500TG SR, Incromega™ E400200 SR, Incromega™ E4010, Incromega™ DHA700TG SR, Incromega™ DHA700E SR, Incromega™ DHA500TG SR, Incromega™ TG3322 SR, Incromega™ E3322 SR, Incromega™ TG3322, Incromega™ E3322, Incromega™ Trio TG/EE (Croda International PLC, Yorkshire, England); EPAX2050TG, EPAX5500EE, EPAX5500TG, EPAX5000EE, EPAX5000TG, EPAX6000EE, EPAX6000TG, EPAX6000FA, EPAX6500EE, EPAX6500TG, EPAX4510TG, EPAX1050TG, EPAX6015TG/EE, EPAX4020TG, and EPAX4020EE (EPAX is a wholly-owned subsidiary of Norwegian company Austevoll Seafood ASA); Omacor® / Lovaza™ / Zodin® / Seacor® finished pharmaceutical product, K85EE, and AGP 103 (Pronova BioPharma Norge AS); MEG-3® EPA/DHA fish oil concentrates (Ocean Nutrition Canada); DHA FNO "Functional Nutritional Oil" and DHA CL "Clear Liquid" (Lonza); Superba™ Krill Oil (Aker); omega-3 products comprising DHA produced by Martek; Neptune krill oil (Neptune); cod-liver oil products and anti-reflux fish oil concentrate (TG) produced by Mollers; Lysi Omega-3 Fish oil; Seven Seas Triomega® Cod Liver Oil Blend (Seven Seas); Fri Flyt Omega-3 (Vesteralens); and Epadel (Mochida). Those commercial embodiments provide for various omega-3 fatty acids, combinations, and other components as a result of the transesterification process or method of preparation in order to obtain the omega-3 fatty acid(s) from various sources, such as marine, algae, microbial, and plant-based sources.

Examples of synthetic oils include, without limitation Maisine, Labrafac lipophile 1349, Labrafac MC60 and Plurol Oleique.

The lipid may be a crude or a refined oil. As used herein, the term crude oil means an oil that is a mixture of the lipids, as extracted from organisms. As used herein, the term refined oil means an oil that is obtained from crude oil by performing at least one fat/oil refining step selected from the group consisting of a degumming step, a deacidifying step, a decoloring step and a deodorizing step so that off-target substances such as phospholipids and sterols will be removed from the crude oil. A person skilled in the art can distinguish a crude oil from a refined oil by routine analysis.

The composition comprising the compound of the invention may be solubilized or dispersed into the oil, thus providing a solution or a dispersion. One of ordinary skill in the art would be familiar with the range of techniques that can be employed for dispersing or solubilizing a drug in an oil. For example, the compound of the invention may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art.

Thus, according to the above, the composition may be a solution or a dispersion. Likewise, oils can also include micelles, microemulsions, macroemulsions, and liposomes.

In some embodiments, the vehicle of the delivery system encapsulates the compound of the invention. The terms "encapsulate" and "entrap" are used herein interchangeably and refer to the incorporation or association of a substance or molecule (e.g., a bioactive compound) in or with an oil. For example, in those embodiments in which the oil is a liposome, the substance or molecule can be associated with the lipid bilayer. The compound can also be encapsulated within micelles, a microemulsion, or a macroemulsion.

The term "micelles" refers to colloidal aggregates of amphipathic molecules that are formed at a well-defined concentration known as the critical micelle concentration. Micelles are oriented with the nonpolar portions of the lipid molecules at the interior of the micelle and the polar portions at the exterior surface, exposed to water. The typical number of aggregated molecules in a micelle (aggregation number) has a range from about 50 to about 100.

Liposomes are self-assembling, substantially spherical vesicles comprising a lipid bilayer that encircles an aqueous interior or core, wherein the lipid bilayer comprises amphipathic lipids having hydrophilic headgroups and hydrophobic tails, in which the hydrophilic headgroups of the amphipathic lipid molecules are oriented toward the aqueous solution, while the hydrophobic tails orient toward the interior of the bilayer. The lipid bilayer structure thereby comprises two opposing monolayers that are referred to as the "inner leaflet" and the "outer leaflet," wherein the hydrophobic tails are shielded from contact with the surrounding medium. The "inner leaflet" is the monolayer wherein the hydrophilic

head groups are oriented toward the aqueous core of the liposome. The "outer leaflet" is the monolayer comprising amphipathic lipids, wherein the hydrophilic head groups are oriented towards the exterior surface of the liposome. Liposomes typically have a diameter ranging from about 25 nm to about 1 μm. The term "liposome" encompasses both multilamellar liposomes comprised of anywhere from two to hundreds of concentric lipid bilayers alternating with layers of an aqueous phase and unilamellar vesicles that are comprised of a single lipid bilayer.

The liposomes contain one or more lipids. The lipids can be neutral, anionic or cationic lipids at physiologic pH.

As used herein, the term "emulsion" refers to a mixture of two immiscible liquids wherein one liquid forms a continuous phase within which droplets of the other liquid are dispersed as a discontinuous phase.

As mentioned, an "emulsion" is a heterogeneous system, consisting of at least two immiscible liquids or phases in the presence of active surface materials such as surfactants or emulgents (emulsifiers).

Within the context of the present invention, the term emulsion encompasses microemulsions and macroemulsions.

As described herein, microemulsions are essentially swollen micelles, although not all micellar solutions can be swollen to form a microemulsion. Microemulsions are thermodynamically stable, are formed spontaneously, and contain particles that are extremely small. Droplet diameters in microemulsions typically range from about 10 to about 100 nm. In contrast, the term macroemulsions refers to droplets having diameters greater than about 100 nm.

Macroemulsions (also generally referred as emulsions) are thermodynamically unstable systems with particle sizes ranging from 1 to 100 μm (orders of magnitude), which, most often, do not form spontaneously. The emulsion consists of droplets of a dispersed phase in the continuous phase. Macroemulsions scatter light effectively and therefore appear milky, because their droplets are greater than a wavelength of light Simple emulsions are classified as water in oil (W/O) or oil in water (O/W) depending on which phase constitutes the disperse phase. An important aspect in the preparation of emulsions from both fundamental and technological points of view is to obtain a desired droplet size and a narrow size distribution. Surfactants (as the main emulsifiers) are used to reduce the interfacial tension between the two phases, and induce emulsion stability for a useful amount of time. Emulsions can be stabilized otherwise with other emulsifiers such as polymers, solid particles or proteins.

In a preferred embodiment, the emulsion is an oil in water emulsion (O/W). In O/W emulsions, droplets of oil are dispersed in the aqueous phase. In the context of the present invention, the principal oil component of the emulsion is the compound of the invention. According to certain embodiments, the oil emulsion of the present invention is oil in water emulsion having an average droplet size of below 1.0 micron. According to certain typical embodiments, the emulsion has an average droplet size of from about 30 nm to 500 nm.

An oil-in-water emulsion (O/W) is a dispersion of oil droplets or colloidal particles in an aqueous medium, with the colloid particles having an oily core surrounded by an interfacial film of emulsifiers and surface acting agents or surfactants. According to the teachings of the present invention, the principal ingredient of the oil component of the emulsion is the compound of the invention.

In another embodiment, the emulsion is a multiple emulsion. In another embodiment the emulsion is an O/W/O emulsion.

According to the present invention, an O/W/O emulsion is a multiple emulsion in which an aqueous phase (hydrophilic) separates the internal and external oil phases. Internal and external phases may or may not be identical. Multiple emulsions are complex polydispersed systems where both oil-in-water and water-in-oil emulsion exist simultaneously which are stabilized by lipophilic and hydrophilic surfactants respectively. The ratio of these surfactants is important in achieving stable multiple emulsions. Multiple emulsions are also called as emulsions of emulsions or double or triple emulsions, since the internal phase itself contains disperse globules, which are miscible with the continuous phase. These are also called as liquid membrane systems because the two miscible phases are separated by an immiscible phase (liquid membrane) which acts as a thin semi-permeable film through which solutes must diffuse in order to traverse from one phase to another.

Any emulsifier and/or surfactant that do not interfere with the activity of the compound of the invention and with the biocompatible characteristics of the emulsion can be used according to the teachings of the present invention. Furthermore, a surfactant which can solubilize the oil within micelles is also suitable for the delivery system.

"Surfactant" refers to amphiphilic compounds generally recognized in the art as having surface active qualities. Surfactants generally include anionic, cationic, nonionic, and zwitterionic compounds. As further used herein, an emulsifier is equivalent to a surfactant.

A surfactant may, for example, lower the surface tension of a liquid or the surface tension between two liquids. For example, surfactants according to the present disclosure may lower the surface tension between the fatty acid oil mixture and an aqueous solution. Chemically speaking, surfactants are molecules with at least one hydrophilic part and at least one hydrophobic (i.e., lipophilic) part. Surfactant properties may be reflected in the hydrophilic-lipophilic balance (HLB) value of the surfactant, wherein the HLB value is a measure of the degree of hydrophilic versus lipophilic properties of a surfactant. The HLB value normally ranges from 0 to 20, where a HLB value of 0 represents high hydrophilic character, and a HLB of 20 represents high lipophilic character. Surfactants are often used in combination with other

surfactants, wherein the HLB values are additive. The HLB value of surfactant mixtures may be calculated as follows:

$$HLBA\ (\text{fraction of surfactant A}) + HLBB\ (\text{fraction of surfactant B}) = HLBA{+}B\ \text{mixture}$$

Surfactants are generally classified as ionic surfactants, e.g., anionic or cationic surfactants, and nonionic surfactants. If the surfactant contains two oppositely charged groups, the surfactant is named a zwitterionic surfactant. Other types of surfactants include, for example, phospholipids.

In at least one embodiment of the present disclosure, the composition comprises at least one surfactant chosen from nonionic, anionic, cationic, and zwitterionic surfactants.

In a preferred embodiment, the surfactant is selected from the group consisting of polysorbates, sorbitan esters, PEO-PPO-block copolymers, POE alkyl ethers, POE castor oil, POE hydrogenated castor oil, POE stearate, POE vitamin E, sucrose esters, alkyl polyglucosides, polyglycolyzed glycerides, polyether alcohols, phospholipids, alkyl sulphates.

Other non-limiting examples of surfactants suitable for the present disclosure are mentioned below.

According to the present disclosure, other exemplary nonionic surfactants include, but are not limited to, diacetyl monoglycerides, diethylene glycol monopalmitostearate, ethylene glycol monopalmitostearate, glyceryl behenate, glyceryl distearate, glyceryl monolinoleate, glyceryl mono-oleate, glyceryl monostearate, macrogol cetostearyl ether such as cetomacrogol 1000 and polyoxy 20 cetostearyl ether, macrogol 15 hydroxystearate, macrogol lauril ethers such as laureth 4 and lauromacrogol 400, macrogol monomethyl ethers, macrogol oleyl ethers such as polyoxyl 10 oleyl ether, macrogol stearates such as polyoxyl 40 stearate, menfegol, mono and diglycerides, nonoxinols such as nonoxinol-9, nonoxinol-10 and nonoxinol-1 1 , octoxinols such as octoxinol 9 and oxtoxinol 10, polyoxamers such as polyoxalene, polyoxamer 188, polyoxamer407, polyoxyl castor oil such as polyoxyl 35 castor oil, polyoxyl hydrogenated castor oil such as polyoxyl 40 hydrogenated castor oil, propylene glycol diacetate, propylene glycol laurates such as propylene glycol dilaurate and propylene glycol monolaurate. Further examples include propylene glycol monopalmitostearate, quillaia, sorbitan esters, and sucrose esters.

Anionic surfactants suitable for the present disclosure include, for example, salts of perfluorocarboxylic acids and perfluorosulphonic acid, alkyl sulphate salts such as sodium dodecyl sulphate and ammonium lauryl sulphate, sulphate ethers such as sodium lauryl ether sulphate, and alkyl benzene sulphonate salts.

Cationic surfactants suitable for the present disclosure include, for example, quaternary ammonium compounds such as benzalkonium chloride, cetylpyridinium chlorides, benzethonium chlorides, and cetyl trimethylammonium bromides or other trimethylalkylammonium salts.

Zwitterionic surfactants include, but are limited to, for example dodecyl betaines, coco amphoglycinates and cocamidopropyl betaines.

In some embodiments of the present disclosure, the surfactant may comprise a phospholipid, derivative thereof, or analogue thereof. Such surfactants may, for example, be chosen from natural, synthetic, and semisynthetic phospholipids, derivatives thereof, and analogues thereof. Exemplary phospholipids surfactants include phosphatidylcholines with saturated, unsaturated and/or polyunsaturated lipids such as dioleoylphosphatidylcholine, dipentadecanoylphosphatidylcholine, dilauroylphosphatidylcholine, dimyristoylphosphatidylcholine, dipalmitoylphosphatidylcholine, distearoylphosphatidylcholine, di-eicopentaenoyl(EPA)choline, didocosahexaenoyl(DHA)choline, phosphatidylethanolamines, phosphatidylglycerols, phosphatidylserines and phosphatidylinositols. Other exemplary phospholipid surfactants include soybean lecithin, egg lecithin, diolelyl phosphatidylcholine, distearoyl phosphatidyl glycerol, PEG-ylated phospholipids, and dimyristoyl phosphatidylcholine.

According to certain embodiments, the emulsifier is one or more selected from the group consisting of polyoxyethylene polyoxypropylene copolymer (commercial name: Poloxamer™), transesterification product of natural vegetable oil triglyceride and polyalkylene polyol (commercial name: Labrafil M 2125™, Labrafil M 1944CS™ Labrasol™), glycerol fatty acid ester (commercial name: Plurol™oleique), Polyoxyl 40 Hydrogenated Castor Oil (commercial name: polyethylene glycol alkyl ether (commercial name: Brij™), polyoxyethylene sorbitan triglyceride ester (commercial name: Tween™), sorbitan fatty acid ester (commercial name: Span Cremophor RH 40); vitamin E polyethyleneglycol succinate (Vitamin E polyethylene glycol succinate), lecithin, sodium lauryl sulfate, and bile acid and derivatives thereof.

Without wishing to be bound by any specific theory or mechanism of action, the higher efficacy observed for oil in water emulsions of the compound of the invention may be attributed to an improved bioavailability of the emulsified oil. The improved bioavailability may be the result of increased solubility of the active component, improved stability during passage of the gastrointestinal tract, improved absorbance from the gastrointestinal tract, improved uptake by the cells or combinations thereof.

According to certain typical embodiments, the composition of the invention, either as an oil dilution, dispersion or emulsion, is formulated for oral or parenteral administration. However, other forms of administration, including transdermal administration are also encompassed within the scope of the present invention.

For oral administration, an effective amount of the composition can be administered in a form selected from,

but not limited to, a solid, semi-solid or liquid state. Specific examples include tablet, capsule, powder, granule, solution, suspension and syrup agents.

Administering the composition of the invention as salts or esters thereof in a solid dosage form is highly desirable in terms of subject convenience and compliance. Capsules for delivery into the gastrointestinal tract are known in the art, and include capsules for oral or rectal administration. Typically, the capsules are so designed as to be dissolved in the water environment of the gastrointestinal tract.

In a particular embodiment, the emulsion is a microemulsion.

In another particular embodiment, the emulsion is a self-emulsifying system.

Self-emulsifying systems are defined as isotropic mixtures of oils, surfactants and cosolvents. Self-emulsifying formulations distribute readily in the gastrointestinal tract. The aqueous contents of the stomach and the digestive motility of the intestines provide the required environment and sufficient agitation for the spontaneous formation of emulsions. Formulation of composition as self-emulsifying systems thus enables avoiding the use of aqueous phase and its formulation as capsules for oral delivery.

The ease of manufacture and scale-up is an additional important advantage of self-emulsifying systems compared with other drug delivery systems, such as solid dispersions, liposomes and nanoparticles. Self-emulsifying systems require very simple and economical manufacturing facilities, such as a simple mixer with an agitator and volumetric liquid filling equipment for large-scale manufacturing.

According to typical embodiments, the Self-emulsifying systems comprises the compound of the invention and at least one emulsifying agent, a surfactant or a combination thereof.

Within the context of the present invention, the term "self-emulsifying system" includes "self-microemulsifying drug delivery system " (SMEDDS) or "self-emulsifying drug delivery system " (SEDDS). SMEDDS and SEDDS refer to a composition that contains an active agent herein defined in intimate admixture with pharmaceutically acceptable excipients such that the system is capable of dissolving the active agent to the desired concentration and producing colloidal structures by spontaneously forming a microemulsion when diluted with an aqueous medium, for example water, or in gastric juices. The colloidal structures can be solid or liquid particles including droplets and nanoparticles. In a SEDDS or SMEDDS system the type of microemulsion produced will be either clear or turbid depending on drug loading and the type of surfactant used.

SEDDS are isotropic mixtures of oils, hydrophobic surfactants and sometimes, containing co-solvents that rapidly produce fine o/w emulsions upon gentle agitation or digestive motility that would be encountered in the gastrointestinal tract. SMEDDS are isotropic mixtures of oil, hydrophilic surfactant and co-solvents that rapidly form O/W microemulsion upon gentle agitation followed by dilution in an aqueous media that will be experienced in the gastrointestinal tract. The basic difference between SEDDS and SMEDDS is that SEDDS typically produce opaque emulsions with a droplet size above 300 nm while SMEDDS form transparent microemulsions with a droplet size of less than 250 nm. In addition, concentration of oil in SMEDDS is less than 20% as compared to 40-80% in SEDDS.

In an embodiment, SEDDS is prepared using surfactants of HLB512. In another embodiment, SMEDDS is prepared with surfactants of HLB412.

Methods of formulation for oral administrations are known to a person skilled in the art. For example, to formulate the composition into tablets, capsules, powders, granules, solutions or suspensions, the compound of the invention, either combined with an oil or emulsions comprising the same or the self emulsifying composition described hereinabove is preferably mixed with a binder, a disintegrating agent and/or a lubricant. If necessary, the resultant composition may be mixed with a diluent, a buffer, an infiltrating agent, a preservative, a penetration enhancer and/or a flavor, using known methods. Examples of the binder include crystalline cellulose, cellulose derivatives, cornstarch, and gelatin. Examples of the disintegrating agent include cornstarch, potato starch, and sodium carboxymethylcellulose. Examples of the lubricant include talc and magnesium stearate. Further, additives such as lactose and mannitol may also be used.

The composition according to the invention may also contain at least one usual excipient or additive in the case of an oily composition, for example a co-solvent, such as for example an alcohol, or an antioxidant.

Said co-solvent, such as for example an alcohol, may be present in the composition for example at a rate of 0 to 5%, in particular 1 to 5% (v/v) of the total volume of the composition.

In a particular embodiment, the composition additionally comprises an antioxidant.

"Antioxidants" are described simplistically as compounds (e.g., enzymes, organic molecules) that slow the rate of oxidation reactions or that can counteract the damaging effects of oxygen. Although the term technically applies to molecules reacting with oxygen, it is often applied to molecules that protect from any free radical (i.e., a molecule with an unpaired electron, such as hydroxyl radicals, lipid oxyl or peroxyl radicals, singlet oxygen, and peroxynitrite formed from nitrogen oxide (NO)). Free radicals are natural by-products of cellular processes in an organism or are created by exposure to environmental factors. Within cellular organisms, free radicals can cause cellular and tissue damage, which can ultimately lead to disease. Antioxidants neutralize free radicals by donating one of their own electrons to the free radical, since the radicalized antioxidant molecule is more stable as a free-radical than the original free-radical.

The antioxidant may be selected from the compounds that are usual in this field, for example: a hydroxylated

substance such as citric acid and derivatives thereof, in particular sodium citrate, calcium citrate or potassium citrate; ascorbic acid and derivatives thereof, in particular isoascorbic acid, sodium ascorbate or calcium ascorbate; a thiol derivative such as for example cysteine, acetylcysteine, dithiothreitol; an ethylenically unsaturated substance, such as sorbic acid, etc.

In a particular embodiment, the antioxidant is selected from the group comprising alpha-tocopherol, ascorbic acid, ascorbyl palmitate, butylated hydroxyanisole (BHA), butylated hydroxytoluene (BHT), citric acid, edetate calcium disodium/edetate disodium, fumaric acid, malic acid, propyl gallate, tert-butylhydroquinone (TBHQ), hydroquinone, monothioglycerol, sodium bisulfite, sodium metabisulfite, sodium sulfite or combinations thereof.

Said antioxidant may be present in the composition for example at a rate of 0.005 to 1% (w/v), of 0.01% to 0.5% (w/v), of 0.025 to 0.25% (w/v), of 0.05 to 0.2% (w/v), of 0.1% to 0.2% (w/v).

A preferred antioxidant is selected from ascorbic acid and derivatives thereof.

In another embodiment, when the vehicle is an emulsion, then, it additionally comprises a co-surfactant.

Thus, as mentioned above, when the vehicle is an emulsion it already comprises a surfactant, therefore, according to this embodiment, the emulsion would comprise a first surfactant and a second surfactant or co-surfactant, being the first and the second surfactants different from each other. As mentioned above, surfactants are often used in combination with other surfactants, wherein the HLB values are additive.

In an embodiment the co-surfactant is selected from the group consisting of short-chain alcohols, alkane diol and triols, PEG, glycol ethers, pyrrolidine derivatives, bile salts, organic acids and salts.

Suitably, when there are 2 surfactants present, each surfactant is present in an amount from about 0.5% to about 5% by weight, based on the total weight of the composition. In another embodiment, there are 3 surfactants present in the formulation. Suitably, when there are 3 surfactants present, each surfactant is present in an amount from about 0.5% to about 5% by weight, based on the total weight of the composition. Similarly, if there are 4 or more surfactants present, they are each present in an amount from about 0.5% to about 5% by weight, based on the total weight of the composition.

The compositions according to the invention may preferably be in a form suitable for administration by the oral route, in particular in liquid form, such as for example oral solution, oral suspension, drops, etc., or in encapsulated form, such as a capsule encapsulating said liquid formulation, in particular a hard or soft capsule based on gelatin.

One of ordinary skill in the art would be familiar with the range of techniques that can be employed for dispersing a drug in an oil. For example, the compound of formula (I) may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art. The dispersion may or may not result in the formation of liposomes.

In the compositions of the present invention, the compound of formula (I) is combined with one or more lipids and an aqueous solvent. Any concentration or amount of compound for dispersion in the lipid is contemplated by the present invention. For example, in certain embodiments the ratio of compound of formula (I):lipid (wt:wt) may be 1:5, 1:6, 1:7, 1:8, 1:9, 1:10, 1:11, 1:12, 1:13, 1:14, 1:15, 1:20, 1:25, 1:30, 1:35, 1:40, 1:45, 1:50, or any ratio derivable therein.

In certain embodiments, the lipid or lipids of the present compositions may comprise about 1%, about 2%, about 3%, about 4% about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, or any range derivable therein, of the present compositions by weight or volume. Thus, it is contemplated that the lipid or lipids of the present composition may comprise any of the lipids, lipid types or other components in any combination or percentage range. In the composition of the present invention, the compound of formula (i) is combined with one or more lipids and an aqueous solvent.

Any concentration or amount of the compound of formula (I) for dispersion in the vehicle is contemplated by the present invention. For example, in certain embodiments the ratio of compound:lipid (wt:wt) may be 1:5, 1:6, 1:7, 1:8, 1:9, 1:10, 1:11, 1:12, 1:13, 1:14, 1:15, 1:20, 1:25, 1:30, 1:35, 1:40, 1:45, 1:50, or any ratio derivable therein.

In preferred embodiment, the ratio of compound of formula (I) (expressed in milligrams) to lipid vehicle (expressed in milliliters) is from 0.01:1 to 200:1, from 0.01:1 to 150:1, from 0.01:1 to 50, preferably from 0.1:1 to 50:1, or from 0.1:1 to 5:1.

More preferably, the ratio of compound of formula (i) (expressed in milligrams) to lipid vehicle (expressed in milligrams) is from 0.01:1 to 200:1, from 0.01:1 to 150:1, from 0.01:1 to 100:1, more preferably from 0.1:1 to 5:1.

In another embodiment, the concentration of the compound of formula (I) is from 0.01 to 200 mg/mL, preferably from 0.01 to 150 mg/mL, preferably from 0.01 to 50 mg/mL, preferably from 0.1 to 50 mg/mL, more preferably from 0.1 to 5 mg/mL and/or wherein the concentration of the compound according to formula (I) in the composition is from 0.01 to 200 mg/mL, preferably from 0.01 to 150 mg/mL, preferably from 0.01 to 50 mg/mL, preferably from 0.1 to 50 mg/mL, more preferably from 0.1 to 5 mg/mL.

In another embodiment, the composition of the invention comprises at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or more of the compound of formula (I) with respect to the total weight of carotenoids in the composition (all values referred by wt%).

The term "carotenoid", as used herein, refers to organic pigments which are structurally composed of a polyene hydrocarbon chain, and which may terminate in a ring. Carotenoids are divided into two classes, xanthophylls (which contain oxygen atoms) and carotenes (which contain no oxygen atoms).

In another embodiment, the composition is in liquid form. It will be understood that the composition may be liquid or solid depending on the temperature. Accordingly, in one embodiment, the composition is liquid at room temperature. As used herein, room temperature is understood as a temperature of 20-22 °C, of around 20 °C or of between 15 to 25 °C.

*A foodstuff, nutritional supplement, cosmeceutical, cosmetic composition or nutraceutical*

Another aspect relates to a foodstuff, a nutritional supplement, a cosmeceutical, a cosmetic composition or a nutraceutical comprising a composition according to the invention.

As used herein, the term "food" is any substance or product of any nature, solid or liquid, natural or processed which due to its characteristics, applications, components, preparation and state of preservation, can usually or ideally be used for some of the following purposes: a) as normal nutrition for human beings or animals or as pleasurable foods; or b) as dietetic products, in special cases of human or animal food (feed). The term "feed" includes all the natural materials and finished products of any origin which, separately or conveniently mixed with one another, are suitable as animal food.

A ready-to-eat food is that which does not need to be diluted by means of an aqueous solution suitable for consumption for example. In principle, the ingredients present in a ready-to-eat food are balanced and there is no need to add additional ingredients to the food to make it ready to eat, such considered by a person skilled in the art. A concentrated food is that in which one or more ingredients are present at a higher concentration than in a ready-to-eat food, therefore for use it is necessary to dilute it by means of an aqueous solution suitable for consumption for example. Non-limiting, illustrative examples of foods provided by this invention include both dairy products and derivatives, for example, fermented milks, yogurt, kephir, curd, cheeses, butters, ice creams, milk-based desserts, etc., and non-dairy products, such as baked products, cakes and pastries, cereals, chocolates, jams, juices, other fruit derivatives, oils and margarines, prepared dishes, etc.

As used herein, the term "cosmeceutical product" refers to a product suitable for use in the body or animal body comprising one or more cosmeceutical products (functional cosmetics, dermaceuticals or active cosmetics), i.e., topical hybrid products with cosmetic-pharmaceutical characteristics containing active ingredients having effect on user's skin, hair and/or nails, at higher and more effective concentrations, therefore they are located in an intermediate level between cosmetic and drug. Illustrative examples of cosmeceutical products include essential oils, ceramides, enzymes, minerals, peptides, vitamins, etc.

As used herein, the term "nutraceutical product" refers to a product suitable for use in human beings or animals, comprising one or more natural products with therapeutic action which provide a health benefit or have been associated with disease prevention or reduction, and it includes dietary supplements presented in a non-food matrix (e.g., capsules, powder, etc.) of a concentrated natural bioactive product usually present (or not) in the foods and which, when taken in a dose higher than that existing in those foods, exerts a favorable effect on health which is greater than effect which the normal food may have. Therefore, the term "nutraceutical product" includes isolated or purified food products as well as additives or food supplements which are generally presented in dosage forms normally used orally, for example, capsules, tablets, sachets, drinkable phials, etc.; such products provide a physiological benefit or protection against diseases, generally against chronic diseases. If desired, the nutraceutical product provided by the invention can contain, in addition to the xanthophylls, one or more nutraceuticals (products or substances associated with disease prevention or reduction), for example, flavonoids, omega-3 fatty acids, etc., and/or one or more prebiotics (non-digestible food ingredients which stimulate probiotic activity and/or growth), for example, oligofructose, pectin, inulin, galacto-oligosaccharides, lactulose, human milk oligosaccharides, dietary fiber, etc.

As used herein, the term "nutritional composition" of the present invention relates to a food product that beneficially affects one or more functions of the body, so as to provide better health and wellness. Accordingly, such a

nutritional composition may be intended for the prevention and/or treatment of a disease or a disease causing factor. Therefore, the term "nutritional composition" of the present invention can be used as a synonym for functional food or foods for particular nutritional purposes, or medical food. A nutritional composition is similar to that of a conventional food and consumed as part of a normal diet.

The term "cosmetic composition" or "personal care composition", as used herein, refers to a composition suitable for use in personal hygiene of human beings or animals, or in order to enhance the natural beauty or change the body appearance without affecting the structure or functions of the human or animal body, comprising one or more products providing such effects. If desired, the cosmetic composition provided by the invention can contain, in addition to the combination of the invention, one or more cosmetics or cosmetic products, i.e., substances or mixtures intended to be placed in contact with the external parts of the human or animal body (e.g., epidermis, hair system, nails, lips, etc.) or with the teeth and the buccal mucosa, for the exclusive or main purpose of cleaning them, perfuming them, changing their appearance, protecting them, keeping them in good condition or correcting body odors. Illustrative examples of cosmetically acceptable vehicles include the products contained in the INCI (International Nomenclature of Cosmetic Ingredients) list. Cosmetic or personal care compositions include products such as balms, pads, pomades, creams, etc. oils, surfactants, humectants, botanical extracts, vitamins, antioxidants, sunscreen agents, perfumes, preservatives, and the like. Illustrative examples of humectants, botanical extracts, vitamins, antioxidants and sunscreen agents.

The ingredients as described hereinabove are preferably provided in a cosmetic composition that may be formulated into a cream, gel, lotion, oil, ointment, powder, stick, cake, or other forms that can be topically applied. The resulting cosmetic composition may be in the form of a liquid, solid, semi-solid, dispersion, suspension, solution or emulsion, and it can be either aqueous-based or anhydrous. The cosmetic compositions of the invention may also be in the form of color cosmetic compositions, such as foundation makeup, mascara, lip color, blush, eye shadow, and the like. Certain other derivatives are lipophilic in nature and will more likely be found in the oil phase of the emulsion. The combination of the invention is preferably found in the water phase of the emulsion or encapsulated in an aqueous phase within liposomes.

The term "cosmetic effective amount", as used herein, relates to the sufficient amount of a compound (i.e. of the combination of the invention) to provide the desired effect and it will generally be determined, by among other causes, the characteristics of the compound itself and the cosmetic effect to be achieved. The dosage for obtaining a cosmetic effective amount it will also depend on a range of factors, such as, for example, age, weight, sex or tolerance of the animal, preferably a mammal and more preferably human.

In a particular embodiment of the invention, the cosmetic composition of the invention is administered by topical route.

If desired, the cosmetic composition of the invention is incorporated in a fabric, a non-woven fabric or a medical device. Illustrative examples of said fabric, non-woven fabric or medical device include but are not limited to bandages, gauzes, t-shirts, panty hose, socks, underwear, girdles, gloves, diapers, sanitary napkins, dressings, bedspreads, towelettes, adhesive patches, non-adhesive patches, occlusive patches, microelectric patches and face masks.

## Non-therapeutic methods

In a third aspect, the invention relates to a non-therapeutic method for improving skin health comprising the step of administering to a patient in need of improved skin health a composition according to the first aspect of the invention or the foodstuff, nutritional supplement, cosmeceutical, cosmetic composition or nutraceutical according to the second aspect of the invention.

In the context of this invention "skin" is understood to be the layers which comprise it, from the uppermost layer or stratum corneum to the lowermost layer or hypodermis, both inclusive. These layers are composed of different types of cells such as keratinocytes, fibroblasts, melanocytes and/or adipocytes among others. In the context of this invention, the term "skin" includes the scalp. The term "skin" includes human skin.

As used herein, "improved skin health" means achieving a measurable improvement of skin quality. The term "skin quality" refers to skin properties such as skin hydration (e.g. 1mprovement of dryness), or skin texture (e.g. skin thickness, roughness, scaliness). For example, with respect to skin texture, the invention includes methods of reducing skin roughness, improving scaliness, and/or improving skin thickness in a subject in need thereof. A person of skill in the art will select the known methods of measuring the improvement of skin quality.

In an embodiment, the method for improving skin health includes at least one treatment selected from the group consisting of protecting the tissues of the skin against ageing, the prevention or treatment of sensible, dry or reactive skins, for improving skin density or firmness, and for increasing skin photoprotection.

In the context of this invention, the term "aging" refers to the changes experienced by the skin with age (chronoaging) or through exposure to the sun (photoaging) or to environmental agents such as tobacco smoke, extreme climatic conditions of cold, heat, or wind, chemical contaminants or pollutants, and includes all the external visible and/or perceptible changes through touch, such as and not restricted to, the development of discontinuities on the skin such as

wrinkles, fine lines, furrows, irregularities or roughness, increase in the size of pores, loss of elasticity, loss of firmness, loss of smoothness, loss of the capacity to recover from deformation, sagging of the skin such as sagging cheeks, the appearance of bags under the eyes or the appearance of a double chin, among others, changes to the color of the skin such as marks, reddening, bags under the eyes or the appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and present the same physical characteristics as aging, such as and not restricted to, flaccidity, sagging, changes to the color or irregularities in the pigmentation, abnormal and/or excessive keratinization. The changes of the skin due to aging (e.g. chronoaging, photoaging and/or environmental aging) are also referred to herein as the symptoms or signs of skin aging.

In another embodiment, the composition is administered topically.

Medical uses

The utility of ACX for treating and/or preventing a variety of medical conditions have been described in the prior art, such as cancer (Satomi Y, Anticancer Res. 2017;37(4):1557-62; Méresse S et al., Int J Mol Sci. 2020 Dec 4;21(23); Peng J et al., Mar Drugs. 2011 Oct 10;9(10):1806-28), cardiovascular disease, hypertension, portal hypertension, pulmonary arterial hypertension, hyperglycemia, diabetes (Type 2), insulin resistance, as hepatoprotective and for lipid management related diseases (Peng J et al., Mar Drugs. 2011 Oct 10;9(10):1806-28), metabolic syndrome and diabetes prevention (Maeda H. J Oleo Sci. 2015 Jan 20;64(2):125-32), coronary atherosclerosis, rheumatoid arthritis and as anti-angiogenic (Méresse S et al., Int J Mol Sci. 2020 Dec 4;21(23)), intestinal flora imbalance, glaucoma, diabetic retinopathy, angiogenesis related diseases, hemorrhagic telangiectasia and psoriasis (Xiao H et al., Mar Drugs. 2020 Dec 11;18(12)), as anti-inflammatory, anaphylactic shock, sepsis, cytokine storm ( Su et al., Front Pharmacol., 2019, DOI: 10.3389/fphar.2019.00906), lupus, sarcoidosis, chronic obstructive pulmonary disease (COPD), asthma, primary biliary cholangitis, sclerosing cholangitis, autoimmune hepatitis, inflammatory bowel disease, Crohn disease, multiple sclerosis, ulcerative colitis (Li X et al., Aging (Albany NY). 2021; 13:2655-2667; Yang YP et al., Nat Prod Res. 2020 Jun; 34(12):1791-1795; Zheng J et al., Mar Drugs. 2019;17(10):552; Yang X et al., J Environ Pathol Toxicol Oncol. 2019;38(3):229-238; Takatani N et al., Biochem Biophys Res Commun. 2020 Jul 23; 528(2) 305-310; Su et al., Front Pharmacol., 2019, DOI: 10.3389/fphar.2019.00906), fibrosis (Xiao H et al., Mar Drugs. 2020 Dec 11;18(12); Ma SY et al. Eur J Pharmacol. 2017 Sep 15;811:199-207), as antibacterial (Karpiński TM, Adamczak A. Fucoxanthin-An Antibacterial Carotenoid. Antioxidants (Basel). 2019 Jul 24;8(8)), as neuroprotective agent (Hu L et al. Biomed Pharmacother. 2018 Oct;106:1484-9), gout and kidney stones (Wang et al., Journal of King Saud University - Science. 2020 Apr; 32(3) 1896-1901), senescence-associated disorders (Guvatova Z et al., Mech Ageing Dev. 2020 Jul;189:111260; Chen, S.-J. et al., Mar. Drugs 2021, 19, 114), glucocorticoid induced muscle atrophy (Zhiyin Let al. Biomed Pharmacother. 2021 Apr 14;139:111590), bone health (Walsh PJ et al. Mar Drugs. 2019 Feb 28;17(3)), age related macular degeneration (Chen S-J et al. Mar Drugs. 2021 Feb 18;19(2)), eczema, atopic dermatitis and itch (Natsume C et al. Int J Mol Sci. 2020 Mar 22;21(6)) and thyroid-related disorders (Yang H et al., Biol Trace Elem Res. 2021 May;199(5):1877-1884).

Moreover, ACX esters of formula (I) or (Ia) are prodrugs of ACX, which are spontaneously transformed into ACX and its cis-isomer in the body. Thus, the esters of the present invention are suitable for the treatment and prevention of the same diseases as reported for ACX.

Thus, in a fourth aspect, the invention relates to the composition of the invention for use in medicine or to a pharmaceutical composition comprising the composition of the invention and, optionally, a pharmaceutically acceptable adjuvant. It will be understood that some of the oils and emulsions defined above in the context of the compositions of the invention may also be pharmaceutically acceptable adjuvants and thus, they can act as vehicles for the composition and as pharmaceutically acceptable adjuvants.

In a fifth aspect, the invention relates to the composition according to the invention, for use in the treatment and/or prevention of a disease selected form the group consisting of cancer, a cardiovascular disease, an intestinal flora imbalance, an inflammatory disease, an autoimmune disease, fibrosis, a bacterial infection, a neurological disease, a hyperuricemic-related disease, a senescence-related disorder, a lipid related disease, glucocorticoid induced muscle atrophy, a bone-related disease, an ocular disease, an angiogenic related disease, psoriasis, eczema, atopic dermatitis, and a thyroid-related disorder.

In a particular embodiment,

- the cancer is as gastric, duodenal, liver, hematologic, breast, glioma, bladder, pancreatic, prostate, colorectal, cervical, nasopharyngeal, lung, kidney, metastasis, melanoma, sarcoma, neural, or skin cancer;
- the cardiovascular disease is hypertension, portal hypertension, pulmonary arterial hypertension, hyperglycemia,

type 2 diabetes, insulin resistance, metabolic syndrome or coronary atherosclerosis;

- the inflammatory disease is anaphylactic shock, sepsis or cytokine storm;
- the autoimmune diseases are lupus, sarcoidosis, chronic obstructive pulmonary disease, asthma, primary biliary cholangitis, sclerosing cholangitis, autoimmune hepatitis, inflammatory bowel disease, Crohn's disease, multiple sclerosis and ulcerative colitis;
- the fibrosis is liver fibrosis, renal fibrosis, lung fibrosis, myocardial fibrosis, interstitial fibrosis, IGG4-related fibrosis, sclerodermia or retroperitoneal fibrosis;
- the neurological disease is Parkinson's disease, Alzheimer's disease, dementia, depression or cerebral ischemia;
- the hyperuricemic-related disease is rheumatoid arthritis, gout or kidney stones;
- the lipid related disease is obesity, cholesterol, trigliceridemia, non-alcoholic steatohepatitis or non-alcoholic fatty liver disease;
- the bone related diseases is bone loss;
- the ocular disease, is as glaucoma, diabetic retinopathy or age related macular degeneration; and/or
- the angiogenesis related disease is hemorrhagic telangiectasia.

In another aspect, the invention relates to a method of treatment and/or prevention of a disease selected form the group consisting of cancer, a cardiovascular disease, an intestinal flora imbalance, an inflammatory disease, an autoimmune disease, fibrosis, a bacterial infection, a neurological disease, a hyperuricemic-related disease, a senescence-related disorder, a lipid related disease, glucocorticoid induced muscle atrophy, a bone-related disease, an ocular disease, an angiogenic related disease, psoriasis, eczema, atopic dermatitis, and a thyroid-related disorder, comprising administering to a subject in need thereof the composition of the invention.

The term "prevention", "preventing" or "prevent", as used herein, relates to the administration of a combination according to the invention or of a medicament comprising said combination to a subject who has not been diagnosed as possibly having the disease, but who would normally be expected to develop said disease or be at increased risk for said disease. The prevention intends to avoid the appearance of said disease. The prevention may be complete (e.g. the total absence of a disease). The prevention may also be partial, such that for example the occurrence of a disease in a subject is less than that which would have occurred without the administration of the composition of the present invention. Prevention also refers to reduced susceptibility to a clinical condition.

The term "treatment", as used herein, refers to any type of therapy, which is aimed at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter.

The term "cancer" as used herein, refers to a disease characterized by uncontrolled cell division (or by an increase of survival or apoptosis resistance) and by the ability of said cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. As used herein, the term cancer includes, but is not limited to, the following types of cancer: breast cancer; biliary tract cancer; bladder cancer; brain cancer including glioblastomas, in particular glioblastoma multiforme, and medulloblastomas; cervical cancer; head and neck carcinoma; choriocarcinoma; colon cancer, colorectal cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer, hepatoma; lung cancer, pleural mesothelioma; lymphomas including Hodgkin's disease and lymphocytic lymphomas; neuroblastomas; oral cancer including squamous cell carcinoma; parotid gland cancer; ovarian cancer including those arising from epithelial cells, stromal cells, germ cells and mesenchymal cells; pancreatic cancer; prostate cancer; kidney cancer, suprarenal cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; cervix cancer, endometrial cancer; testicular cancer including germinal tumors such as seminoma, non-seminoma (teratomas, choriocarcinomas), stromal tumors, and germ cell tumors; thyroid cancer including thyroid adenocarcinoma and medullary carcinoma; and renal cancer including adenocarcinoma and Wilms tumor. Other cancers will-be known to one of ordinary skill. In a particular embodiment, the cancer is as gastric, duodenal, liver, hematologic, breast, glioma, bladder, pancreatic, prostate, colorectal, cervical, nasopharyngeal, lung, kidney, metastasis, melanoma, sarcoma, neural, or skin cancer.

In a particular embodiment, the cardiovascular disease is hypertension, portal hypertension, pulmonary arterial hypertension, hyperglycemia, type 2 diabetes, insulin resistance, metabolic syndrome or coronary atherosclerosis.

In a particular embodiment, the inflammatory disease is anaphylactic shock, sepsis or cytokine storm.

In a particular embodiment, the autoimmune diseases is lupus, sarcoidosis, chronic obstructive pulmonary disease, asthma, primary biliary cholangitis, sclerosing cholangitis, autoimmune hepatitis, inflammatory bowel disease, Crohn's disease, multiple sclerosis and ulcerative colitis.

"Fibrosis" as used herein relates to the formation of excess fibrous connective tissue in an organ or tissue in a reparative or reactive process. Although fibrosis may be a benign state, the present invention relates preferably to fibrosis in a pathological state. In a particular embodiment, the fibrosis is liver fibrosis, renal fibrosis, lung fibrosis, myocardial fibrosis, interstitial fibrosis, IGG4-related fibrosis, sclerodermia or retroperitoneal fibrosis.

In a particular embodiment, the neurological disease is Parkinson's disease, Alzheimer's disease, dementia, depression or cerebral ischemia.

In a particular embodiment, the hyperuricemic-related disease is rheumatoid arthritis, gout or kidney stones.

In a particular embodiment, the lipid-related disease is obesity, cholesterol, trigliceridemia, non-alcoholic steatohepatitis or non-alcoholic fatty liver disease.

In a particular embodiment, the bone related disease is bone loss.

In a particular embodiment, the ocular disease is glaucoma, diabetic retinopathy or age-related macular degeneration.

In a particular embodiment, the angiogenesis-related disease is hemorrhagic telangiectasia.

In a further aspect, the invention relates the composition of the invention for use in the treatment and/or prevention of a disease mediated by glucocorticoid receptor activity, a senescence-associated disease or disorder, or as adjuvant in the treatment of cancer with chemotherapy, preferably taxane-comprising chemotherapy.

The term "glucocorticoid receptor", also known as GR, GCR, or NR3C1 (nuclear receptor subfamily 3, group C, member 1), as used herein refers to the receptor to which cortisol and other glucocorticoids bind. The human gene is shown in the Ensembl database under accession number ENSG00000113580 (Ensembl release 106, Apr 2022).

As used herein, the terms "corticoids" or "corticosteroids" refer to a class of steroid hormones that are produced in the adrenal cortex of vertebrates, as well as the synthetic analogues of these hormones. Corticosteroids are involved in a wide range of physiological processes, including stress response, immune response, and regulation of inflammation, carbohydrate metabolism, protein catabolism, blood electrolyte levels, and behavior. There are two types of corticoids, glucocorticoids and mineralocorticoids.

Glucocorticoids are corticosteroids that bind to the glucocorticoid receptor. The structure of glucocorticoids comprises a steroid ring system.

As used herein, the term "cortisol" or "hydrocortisone" refers to a glucocorticoid of formula:

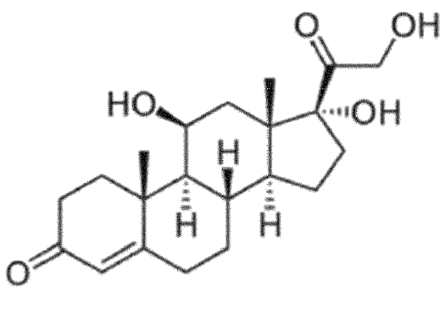

In a particular embodiment, the disease mediated by glucocorticoid receptor activity is selected from the group consisting of cortisol-induced immunosuppression, cortisol-induced insulin resistance, altered skin barrier homeostasis, Cushing's syndrome, subclinical Cushing's syndrome, subclinical hypercortisolemia, metabolic syndrome, inflammatory bowel disease, muscle wasting and muscle dystrophy, insomnia associated to circadian rhythm disorders, hypertension, osteoporosis, water retention, cortisol-induced DNA damage, migraine, psychosis, appetite reduction, depression, stress disorders and cognitive disorders such as Alzheimer's disease catatonia, amyotrophic lateral sclerosis, delirium, post-traumatic stress disorders, impaired memory retrieval, and borderline personality disorder.

In another embodiment, the senescence-associated disorder is fibrosis, particularly wherein the fibrosis is characterized by having a high number of senescent cells, more particularly the fibrosis is lung fibrosis, chronic obstructive pulmonary disease, myocardial fibrosis or renal fibrosis; or wherein the senescence-associated disease is cancer, preferably a hematologic cancer or skin cancer.

Cosmetic methods

The utility of compounds of ACX in preventing and/or decreasing cutaneous senescence and/or for ameliorating the cosmetic adverse effects of aging have been described in the prior art (Peng J et al. Mar Drugs. 2011 Oct 10;9(10):1806-28; Kang SY et al. J Cosmet Sci. 2020;71(2):53-64.). As previously explained, ACX esters of formula (I) or (Ia) are spontaneously transformed into ACX and its cis-isomer in the body. Thus, the esters of the present invention are suitable for their use in the same cosmetic methods as reported for ACX.

Thus, in a last aspect, the invention relates to a cosmetic method for preventing and/or decreasing cutaneous senescence and/or for ameliorating the cosmetic adverse effects of aging comprising administering the composition of the invention, the food, cosmeceutical, nutraceutical or cosmetic composition according to the second aspect, to a subject in need thereof. In a more particular embodiment, the cosmetic method for preventing and/or decreasing cutaneous senescence and/or ameliorating the cosmetic adverse effects of aging comprises the administration of the composition of the invention, wherein the composition comprises the compound of formula (I) or (Ia), preferably of formula (Ia), preferably wherein R is methyl, according to the first aspect or the food, cosmeceutical, nutraceutical or cosmetic composition according to the second aspect, to a subject in need thereof.

As used herein, the term "cosmetic method" relates to a method used to enhance the appearance of the skin in a subject. Cosmetic compositions used in the cosmetic method of the invention include skin-care creams, lotions, powders, lipsticks, eye and facial makeup, towelettes, gels, deodorants, hand sanitizer, baby products, bath oils, bubble baths, bath salts, butters and many other types of products.

Skin aging is a multi-factorial process that affects nearly every aspect of its biology and function; it is driven by both intrinsic (e.g., time, genetic factors, hormones) and extrinsic (e.g., UV exposure, pollution, cigarette smoke) factors. Skin aging is also produced by senescence.

Cellular senescence is a growth arrest that occurs as a result of different damaging stimuli, including DNA damage, telomere shortening and dysfunction or oncogenic stress. Senescent cells exert a pleiotropic effect on development, tissue aging and regeneration, inflammation, wound healing and tumor suppression. Senescent cells are characterized by their inability to proliferate, resistance to apoptosis, and secretion of factors that promote inflammation and tissue deterioration.

Senescent keratinocytes and fibroblasts appear to accumulate with age in human skin. Moreover, senescent cells express genes that have long-range, pleiotropic effects-degradative enzymes, growth factors, and inflammatory cytokines.

"Cosmetic adverse effects of aging", as used herein relates to characteristics of intrinsic or chronological aging and include as a way of illustrative non limitative visible signs such as thin and dry skin, fine wrinkles, decreased elasticity, aberrant pigmentation, hair graying and hair loss.

"Cutaneous senescence", as used herein relates to means the state of growing old and particularly damage to the epidermal cells of human skin which results from partial damage or complete destruction of the cells, conversion of imide bonds to amide bonds in collagen and/or elastin caused by toxic byproducts of oxygen metabolism, free-radical pathology mechanisms or by photo-damage and generalized aging.

The cosmetic method of the invention is intended to decrease epidermal cell and thereby cutaneous senescence in a human by reducing or inhibiting senescence, including one or more affects such as reversing photo-damage or other regenerative effects, such as increasing underlying skin vascularity, increasing the rate of cellular replication and desquamation producing a more youthful appearance, increasing collagen synthesis and homogeneity, delaying cutaneous atrophy and thinning of epidermis and dermis, and the like.

The combination of the invention may be administered in a cosmetically effective amount.

The term "cosmetic effective amount", as used herein, relates to the sufficient amount of a compound or composition of the invention to provide the desired effect and it will generally be determined, by among other causes, the characteristics of the compound itself and the cosmetic effect to be achieved. The dosage for obtaining a cosmetic effective amount it will also depend on a range of factors, such as, for example, age, weight, sex or tolerance of the animal, preferably a mammal and more preferably human.

In a particular and preferred embodiment of the cosmetic method of the invention, the cosmetic composition of the invention is administered by topical route. Adequate formulations for topical administration of the combination of the invention have been detailed in the context of the cosmetic compositions of the invention and equally apply to the cosmetic method of the invention.

If desired, the cosmetic composition of the invention is incorporated in a fabric, a non-woven fabric or a medical device. Illustrative examples of said fabric, non-woven fabric or medical device include but are not limited to bandages, gauzes, t-shirts, panty hose, socks, underwear, girdles, gloves, diapers, sanitary napkins, dressings, bedspreads, towelettes, adhesive patches, non-adhesive patches, occlusive patches, microelectric patches and face masks.

The following examples represent specific embodiments of the present invention. They do not intend to limit in any way the scope of the invention defined in the present description.

Further Aspects Of The Invention

1. A composition comprising a compound of formula (I)

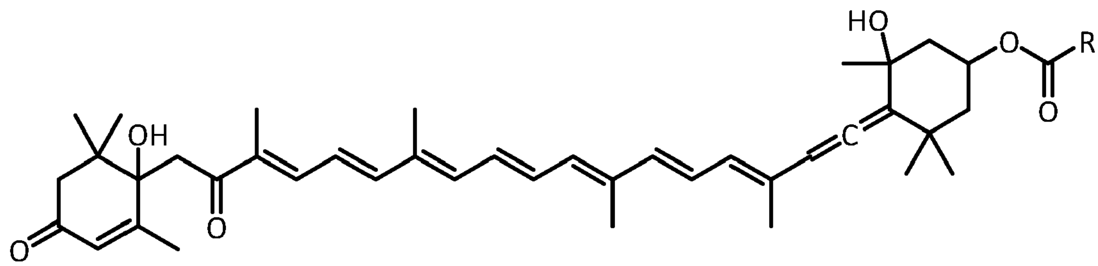

(I)

wherein R is selected from the group consisting of linear or branched $C_1$-$C_4$ alkyl, or a stereoisomer thereof and a vehicle, wherein the vehicle is selected from an oil or an emulsion.

2. The composition according to aspect 1 wherein the compound has formula (Ia)

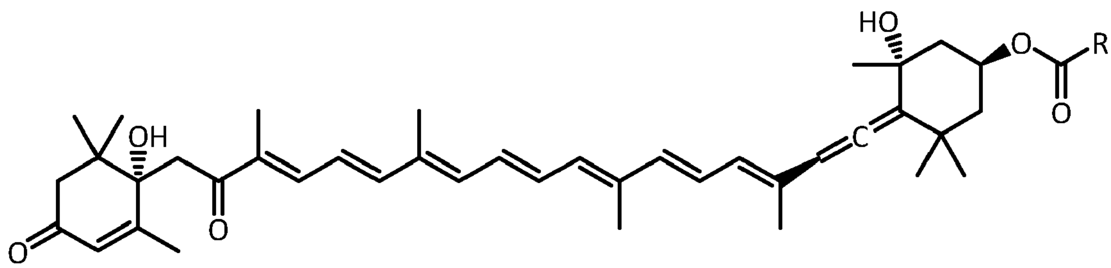

(Ia)

wherein R is as defined in claim 1.

3. The composition according to aspects 1 or 2, wherein R is methyl.

4. The composition according to any of aspects 1 to 3, wherein the vehicle is an oil enriched in polyunsaturated or monounsaturated fatty acids.

5. The composition according to any of aspects 1 to 3, wherein the emulsion is an O/W emulsion, an O/W/O emulsion or a self-emulsifying system.

6. The composition according to any of aspects 1 to 5, wherein the oil is a vegetable oil, a microorganism oil, krill oil or a fish oil.

7. The composition according to aspect 6 wherein the oil is enriched in polyunsaturated fatty acids and/or omega-3 fatty acids.

8. The composition according to aspect 7, wherein the lipid vehicle is a vegetable oil which is selected from the group consisting of sunflower oil, coconut oil, canola oil, cottonseed oil, olive oil, palm oil, rapeseed oil, safflower oil, almond oil, beech nut oil, brazil nut oil, cashew oil, hazelnut oil, macadamia oil, mongongo nut oil, pecan oil, pine nut oil, pistachio oil, walnut oil, pumpkin seed oil, grapefruit seed oil, lemon oil, orange oil, acai oil, black seed oil, blackcurrant seed oil, borage seed oil, evening primrose oil, flaxseed oil, amaranth oil, apricot oil, apple seed oil, argan oil, avocado oil, babassu oil, ben oil, borneo tallow nut oil, cape chestnut oil, carob pod oil, cocklebur oil, cohune oil, coriander seed oil, date seed oil, dika oil, grape seed oil, kapok seed oil, kenaf seed oil, lallemantia oil, mafura oil, marula oil, mustard oil, nige seed oil, okra seed oil, papaya seed oil, perilla seed oil, persimmon seed oil, pequi oil, pili nut oil, pomegranate seed oil, poppy seed oil, pracaxi oil, prune kernel oil, quinoa oil, ramtil oil, rice bran oil, royle oil, sacha inchi oil, sapote oil, seje oil, taraminra oil, tea seed oil, thistle oil, tigernut oil, tomato seed oil, wheat germ oil, peanut oil, sesame oil, soybean oil, corn oil and mixtures thereof; preferably sunflower oil.

9. The composition according to aspect 8, wherein the vegetable oil is sunflower oil.

10. The composition according to aspect 6, wherein the lipid vehicle is a fish oil or a microorganism oil which is enriched in DHA or EPA.

11. The composition according to any of aspects s 1 to 10, additionally comprising an antioxidant.

12. The composition according to any of aspects 1 to 11, wherein, when the vehicle is an emulsion, then, it additionally comprises a surfactant.

13. The composition according to any of the preceding aspects, wherein the ratio of compound of formula (I) (expressed in milligrams) to lipid vehicle (expressed in milliliters) is from 0.01:1 to 200:1, preferably from 0.01:1 to 150:1, preferably from 0.01:1 to 50:1, preferably from 0.1:1 to 50:1 more preferably from 0.1:1 to 5:1.

14. The composition according to any of the preceding aspects, wherein the composition is in liquid form.

15. The composition according to any of the preceding aspects, wherein the concentration of the compound of formula (I) is from 0.01 to 200 mg/mL, from 0.01 to 150 mg/mL, preferably from 0.01 to 50 mg/mL, preferably from 0.1 mg/mL to 50 mg/mL, more preferably from 0.1 to 5 mg/mL and/or wherein the concentration of compound of formula (I) in the composition is from 0.01 to 200 mg/mL, preferably from 0.01 to 150 mg/mL, preferably from 0.01 to 50 mg/mL, preferably from 0.1 mg/mL to 50 mg/mL more preferably from 0.1 to 5 mg/mL.

16. The composition according to any of the preceding aspects, which comprises at least 55 wt % of the compound of formula (I) with respect to the total weight of carotenoids in the composition.

17. A foodstuff, nutritional supplement, cosmeceutical, cosmetic composition or nutraceutical comprising a composition according to any of claims 1 to 8.

18. A non-therapeutic method for improving skin health comprising the step of administering to a patient in need of improved skin health a composition according to any of aspects 1 to 16 or the foodstuff, nutritional supplement, cosmeceutical, cosmetic composition or nutraceutical according to aspect 17.

19. The method according to aspect 18, wherein the method for improving skin health includes at least one treatment selected from the group consisting of protecting the tissues of the skin against ageing, the prevention or treatment of sensible, dry or reactive skins, for improving skin density or firmness, and for increasing skin photoprotection.

20. The method according to aspects 18 or 19 wherein the composition is administered topically.

21. The composition according to any aspects 1 to 16 for use in medicine or a pharmaceutical composition comprising the composition of the invention.

22. The composition according to any one of aspects 1 to 16 for use in the treatment and/or prevention of a disease selected form the group consisting of cancer, a cardiovascular disease, an intestinal flora imbalance, an inflammatory disease, an autoimmune disease, fibrosis, a bacterial infection, a neurological disease, a hyperuricemic-related disease, a senescence-related disorder, a lipid related disease, glucocorticoid induced muscle atrophy, a bone-related disease, an ocular disease, an angiogenic related disease, psoriasis, eczema, atopic dermatitis, and a

thyroid-related disorder.

23. The composition for use according to aspect 22 wherein:

- the cancer is as gastric, duodenal, liver, hematologic, breast, glioma, bladder, pancreatic, prostate, colorectal, cervical, nasopharyngeal, lung, kidney, metastasis, melanoma, sarcoma, neural, or skin cancer;
- the cardiovascular disease is hypertension, portal hypertension, pulmonary arterial hypertension, hyperglycemia, type 2 diabetes, insulin resistance, metabolic syndrome or coronary atherosclerosis;
- the inflammatory disease is anaphylactic shock, sepsis or cytokine storm;
- the autoimmune diseases is lupus, sarcoidosis, chronic obstructive pulmonary disease, asthma, primary biliary cholangitis, sclerosing cholangitis, autoimmune hepatitis, inflammatory bowel disease, Crohn's disease, multiple sclerosis and ulcerative colitis;
- the fibrosis is liver fibrosis, renal fibrosis, lung fibrosis, myocardial fibrosis, interstitial fibrosis, IGG4-related fibrosis, sclerodermia or retroperitoneal fibrosis;
- the neurological disease is Parkinson's disease, Alzheimer's disease, dementia, depression or cerebral ischemia;
- the hyperuricemic-related disease is rheumatoid arthritis, gout or kidney stones;
- the lipid related disease is obesity, cholesterol, trigliceridemia, non-alcoholic steatohepatitis or non-alcoholic fatty liver disease;
- the bone related diseases is bone loss;
- the ocular disease, is as glaucoma, diabetic retinopathy or age related macular degeneration; and/or
- the angiogenesis related disease is hemorrhagic telangiectasia.

24. The composition according to any of aspects 1 to 16, for use in the treatment and/or prevention of a disease mediated by glucocorticoid receptor activity, a senescence-associated disease or disorder, or as adjuvant in the treatment of cancer with chemotherapy, preferably taxane-comprising chemotherapy.

25. The composition for use according to aspect 24, wherein the disease mediated by glucocorticoid receptor activity is selected from the group consisting of cortisol-induced immunosuppression, cortisol-induced insulin resistance, altered skin barrier homeostasis, Cushing's syndrome, subclinical Cushing's syndrome, subclinical hypercortisolemia, metabolic syndrome, inflammatory bowel disease, muscle wasting and muscle dystrophy, insomnia associated to circadian rhythm disorders, hypertension, osteoporosis, water retention, cortisol-induced DNA damage, migraine, psychosis, appetite reduction, depression, stress disorders and cognitive disorders such as Alzheimer's disease catatonia, amyotrophic lateral sclerosis, delirium, post-traumatic stress disorders, impaired memory retrieval, and borderline personality disorder.

26. The composition for use according to aspect 24, wherein the senescence-associated disorder is fibrosis, particularly wherein the fibrosis is characterized by having a high number of senescent cells, more particularly the fibrosis is lung fibrosis, chronic obstructive pulmonary disease, myocardial fibrosis or renal fibrosis; or wherein the senescence-associated disease is cancer, preferably a hematologic cancer or skin cancer.

27. A cosmetic method for preventing and/or decreasing cutaneous senescence and/or for ameliorating the cosmetic adverse effects of aging comprising administering the composition according to any of aspects 1 to 16, or the foodstuff, nutritional supplement, cosmeceutical, cosmetic composition or nutraceutical according to aspect 17, to a subject in need thereof.

## EXAMPLES

### ***Analytical methods***

*Nuclear magnetic resonance (NMR)*

NMR spectra were acquired on a Varian 400 MHz or a 500 MHz instrument. $^1$H chemical shifts are quoted relative to deuterated solvent.

### ***Example 1. Synthesis of amarouciaxanthin A acetate***

Fucoxanthin (1 eq), NMO monohydrate (2 eq) and freshly dried 4Å molecular sieves are dissolved with dichloromethane (DCM). TPAP (0.12 eq) is added at room temperature. When the reaction reaches complete conversion (>97%), $SiO_2$ (30 eq) is added to the reaction. The reaction is stirred at room temperature until complete conversion. The reaction is filtered to remove the $SiO_2$. The filtrate is washed with aqueous 5% $Na_2S_2O_3$. The organic layer is washed twice with brine, dried with $Na_2SO_4$, filtered and evaporated to dryness. The residue is dried using high-vacuum to obtain the desired compound as a red solid.

$^1$H-NMR (400MHz, $CDCl_3$) $\delta$ 7.10 (d, J = 10.9 Hz, 1H), 6.83 - 6.50 (m, 5H), 6.45 (d, J = 11.6 Hz, 1H), 6.35 (d, J = 15.0 Hz, 1H), 6.27 (d, J = 11.7 Hz, 1H), 6.13 (d, J = 11.5 Hz, 1H), 6.05 (s, 1H), 5.84 (s, 2H), 5.44 - 5.33 (m, 1H), 3.05 (d, J = 15.0 Hz, 1H), 2.92 (d, J = 15.0 Hz, 1H), 2.47 (d, J = 18.1 Hz, 1H), 2.38 - 2.24 (m, 2H), 2.04 (s, 3H), 1.99 (m, 7H), 1.95 (s, 3H), 1.90 (d, J = 1.0 Hz, 3H), 1.81 (s, 3H), 1.51 (t, J = 12.2 Hz, 1H), 1.40 (m, 8H), 1.14 - 1.02 (m, 9H).

***Example** 2. **Synthesis of paracentrone acetate***

Amarouciaxanthin A acetate is mixed with water and heated to 70 °C. Once the reaction does not evolve, it is cooled down and extracted with dichloromethane. The organic layer is dried, filtered and evaporated to dryness. The resulting residue is purified by $SiO_2$ column chromatography to obtain the desired compound as a brown solid.

Paracentrone acetate: $^1$H-NMR (500 MHz, $CDCl_3$) $\delta$ 7.14 (dd, J = 10.7, 1.2 Hz, 1H), 6.77 - 6.53 (m, 5H), 6.42 - 6.30 (m, 2H), 6.26 (d, J = 11.5 Hz, 1H), 6.12 (dd, J = 11.4, 1.0 Hz, 1H), 6.06 (d, J = 7.2 Hz, 1H), 5.38 (m, 1H), 2.36 (s, 3H), 2.31 - 2.25 (m, 1H), 2.03 (s, 3H), 2.02 - 1.96 (m, 6H), 1.93 (d, J = 0.9 Hz, 3H), 1.81 (t, J = 3.6 Hz, 3H), 1.50 (m, 1H), 1.44 - 1.39 (m, 1H), 1.36 (d, J = 15.3 Hz, 6H), 1.09 - 1.03 (m, 3H).

***Example 3. Synthesis of amarouciaxanthin A acetate using different oxidants***

The same procedure of example 1 was carried out but replacing TPAP and NMO with IBX, Dess-Martin periodinane and Swern oxidation. The results are gathered in the table below:

| *Reagent* | *Oxidized fucoxanthin* | *One pot $SiO_2$ addition* |
|---|---|---|
| IBX | 100% conversion | Mixture oxidized fucoxanthin + desired compound |
| Dess-Martin periodinane | 55% conversion | - |
| Swern oxidation | 10% conversion | - |
| TPAP | 100% conversion | 100% conversion |

***Example 4. Synthesis of amarouciaxanthin A***

Amarouciaxanthin A acetate (1 eq) and sodium taurocholate (30 eq) are dissolved in MeOH. The solvent is evaporated to dryness. The residue is dissolved in phosphate-buffered saline (PBS, pH 7) and lipase (1340 U/mg compound) is added. The reaction is shaken at 37 °C until maximum conversion (aprox 80%). The reaction is quenched with MeOH and extracted with $Et_2O$ and brine. The aqueous layer is extracted with $Et_2O$ until no colour is observed in the organic layer. The combined organic layers are dried, filtered and evaporated to dryness. The residue is purified by $SiO_2$ column chromatography and amarouciaxanthin A was obtained as a red solid and the unreacted amarouciaxanthin A acetate is recovered.

$^1$H-NMR (500 MHz, acetone) $\delta$ 7.53 (dd, J = 10.8, 1.1 Hz, 1H), 6.93-6.81 (m, 2H), 6.80 - 6.67 (m, 3H), 6.52 (d, J = 11.6 Hz, 1H), 6.36 (dd, J = 17.7, 13.4 Hz, 2H), 6.16 (dd, J = 11.4, 1.0 Hz, 1H), 6.02 (s, 1H), 5.91 (s, 1H), 5.74 - 5.69 (m, 1H), 4.28 - 4.17 (m, 1H), 3.61 (d, J = 1.6 Hz, 1H), 3.48 (d, J = 5.1 Hz, 1H), 3.21 (q, J = 15.5 Hz, 2H), 2.80 (d, J = 1.6 Hz, 3H), 2.78 - 2.75 (m, 1H), 2.62 (d, J = 17.6 Hz, 1H), 2.21 - 2.11 (m, 2H), peaks under acetone, 2.02 (s, 2H), 1.99 (s, 2H), 1.94 (t, J = 3.3 Hz, 2H), 1.91 (m, 1H), 1.89 - 1.83 (m, 5H), 1.40 - 1.24 (m, 7H), 1.07 - 0.98 (m, 6H).

***Example 5. Conversion of ACX-Ac to ACX after oral absorption and bioavailability after oral administration of ACX-Ac, FX and ACX***

It is well described that fucoxanthin (FCX) administered orally has a minimal absorption. Instead, it is thought to be metabolized to fucoxanthinol by intestinal or pancreatic cells and later converted to amarouciaxanthin (ACX) in the liver.

Amarouciaxanthin A acetate (ACX-Ac) is a novel compound that acts as a prodrug for ACX. Unlike FX, there are no intermediate metabolites.

Immunocompetent mice from the strain Hsd:ICR (CD-1) (females, 6-7 weeks old from ENVIGO), were housed

under sterile conditions at a constant temperature of 20-22°C and relative humidity (45-65%) under daily cycles of light/darkness (12 hours). Sterilized water and food were available ad libitum.

ACX-Ac was dissolved at 1 mg/mL in sunflower oil and sterilized by filtration. FCX was dissolved at 2 mg/mL in sunflower oil and sterilized by filtration. Animals were weighted before the assay to determine the appropriate dispensing volume at 5 mL/kg (FCX) or 10 mL/kg (ACX-Ac). Formulations were administered once, intragastrically, at 10 mg/kg. Blood was collected at different time points, as stated in Table 1. It was extracted either through the facial vein or by intracardiac puncture (end points), on recipients containing 5 μL of 0.5 M EDTA.

Blood samples were analyzed by HPLC-MS/MS following proprietary analytical methods and the concentration of ACX in each sample was determined. A non-compartmental pharmacokinetic analysis was carried out using individual plasma concentrations versus sampling times by means of the PKPlus® software version 2.0 (Simulations Plus, Lancaster, CA 93534, USA).

*Table 1. Treatment groups in the FCX oral kinetic assay.*

| Group | Number of animals | Blood extraction |
|---|---|---|
| **1** | 3 | 5 min, 8h |
| **2** | 3 | 15 min, 24h |
| **3** | 3 | 30 min, 48h |
| **4** | 3 | 1h |
| **5** | 3 | 2h |
| **6** | 3 | 4h |

*Table 2. Treatment groups in the ACX-Ac oral kinetic assay.*

| Group | Number of animals | Blood extraction |
|---|---|---|
| **1** | 3 | 2 min, 8h |
| **2** | 3 | 30 min, 24h |
| **3** | 3 | 2h, 48h |
| **4** | 3 | 4h, 72h |
| **5** | 3 | 15 min, 16h |
| **6** | 3 | 1h, 36h |

As described in literature, the oral absorption of fucoxanthin was negligible after administering an oil formulation of this xanthophyll. ACX-Ac was not detected in plasma samples either when administered orally. The absorption of ACX was subsequently analyzed.

The plasma levels of ACX were substantially higher following the administration of ACX-Ac in comparison to those obtained after FX administration, as seen in Figure 1 and Table 3.

*Table 3. Estimated pharmacokinetic constants of ACX after oral delivery of different formulations. A noncompartmental pharmacokinetic analysis was carried out using plasma concentrations of ACX.*

| | **ASSAY 1** | **ASSAY 2** |
|---|---|---|
| **Compound administered** | FX | ACX-Ac |
| **Vehicle** | Sunflower oil | Sunflower oil |
| **Dose** | 10 mg/kg | 10 mg/kg |
| **Route of administration** | Oral | Oral |
| Pharmacokinetic parameters | | |
| **Cmax (μM)** | **2.867** | **9.346** |

(continued)

| | ASSAY 1 | ASSAY 2 |
|---|---|---|
| **AUCt (μM·h)** | **51.19** | **80.59** |
| *Cmax, maximum observed plasma concentration; AUCt, area under the concentration-time curve from 0 to the time of the last quantifiable observation (calculated using the linear-log trapezoidal rule); StDev, statistical deviation.* | | |

***Example 6. Evaluation of ACX, ACX-Ac and FX solubility in different lipid vehicle formulations.***

About 25 mg of compound was weighted into a glass vial and 1 mL of the lipophilic solvent was added to the vial containing the compound. The vial was vortexed for 1 minute followed by sonication for 30 minutes. The resulting solution was visually observed for any undissolved particles.

*Sample preparation*

The sonicated sample was filtered through a 0.22 μm PTFE filter and 500 μL of this filtered sample was dissolved with 25 mL of a EtOH:hexane (80:20) solution ("dilution 1"). 1 mL of "dilution 1" was dissolved with 10 mL of ACN to obtain "dilution 2". 1 mL of "dilution 2" was filtered through a 0.22 μm PTFE filter and directly used for assay by HPLC. The concentration was calculated by extrapolation from a calibration curve.

*Calibration curve*

1.00 mg of standard compound was accurately weighted (precision scale) and diluted to 100 ppm in a volumetric flask. Several concentrations (50, 25, 12.5, 5, 2.5 ppm) were prepared from the initial 100 ppm solution in order to prepare a calibration curve (r > 0.99).

*Table 4. Comparison of lipid vehicle formulations*

| | FCX | ACX-A | ACX |
|---|---|---|---|
| | *solubility (mg/mL)* | *solubility (mg/mL)* | *solubility (mg/mL)* |
| **Labrasol ALF**[1] | 22,3 | 22,4 | 22,4 |
| **Labrafil M 2125**[2] | 23,0 | 23,0 | 20,3 |
| **Maisine**[3] | 23,0 | 22,9 | 19,9 |
| **Labrafac Lipophile 1349**[4] | 16,9 | 21,7 | 19,0 |
| **Plurol Oleique**[5] | 20,7 | 11,9 | 16,7 |
| **Labrafil** M **1944CS**[6] | 17,6 | 22,6 | 19,9 |
| **Sunflower oil**[7] | 5,0 | 19,9 | 9,2 |
| **CMC solution in water**[8] | 0,0 | 0,0 | 0 |
| **DHA (fish oil)**[9] | 4,1 | 18,3 | 7,2 |

(continued)

| | FCX | ACX-A | ACX |
|---|---|---|---|
| | *solubility (mg/mL)* | *solubility (mg/mL)* | *solubility (mg/mL)* |
| **Labrafac MC60**[10] | 20,4 | 21,0 | 15,9 |

[1]Labrasol ALF: SEEDS: (Self-emulsifying drug delivery system, MCT (medium chain triglyceride). Mono-, di- and triglycerides and mainly PEG-8 (MW 400) mono- and diesters of caprylic (C8) and capric (C10) acids. No insaturations.
[2]Labrafil M2125: SEEDS, LCT (Long chain triglyceride). Mono-, di- and triglycerides and PEG-6 (MW 300) mono- and diesters of linoleic (C18:2) acid. With insaturations.
[3]Maisine: LCT, synthetic oil. Mono-, di- and triglycerides of mainly linoleic (C18:2) and oleic (C18:1) acids. With insaturations.
[4]Labrafac Lipophile 1349: MCT, synthetic oil. Medium chain triglycerides of caprylic (C8) and capric (C10) acids. No insaturations.
[5]Plurol Oleique: LCT, synthetic oil. Polyglyceryl-3 esters of oleic acid (C18:1). With insaturations. [6]Labrafil M1944CS: LCT, SEDDS. Mono-, di- and triglycerides and PEG-6 (MW 300) mono- and diesters of oleic (C18:1) acid. With insaturations.
[7]Sunflower oil: LCT, vegetable oil. Triglycerides of linoleic (C18:2) and oleic acid (C18:1). With insaturations.
[8]CMC solution in water: aqueous. Carboxymethyl cellulose.
[9]DHA (fish oil): LCT, PUFA-enriched oil. Omega-3 fatty acids.
[10]Labrafac MC60: MCT, synthetic oil. Mono- and diglycerides of caprylic acid (C8) and capric acid (C10).

## Claims

1. A composition comprising a compound of formula (I)

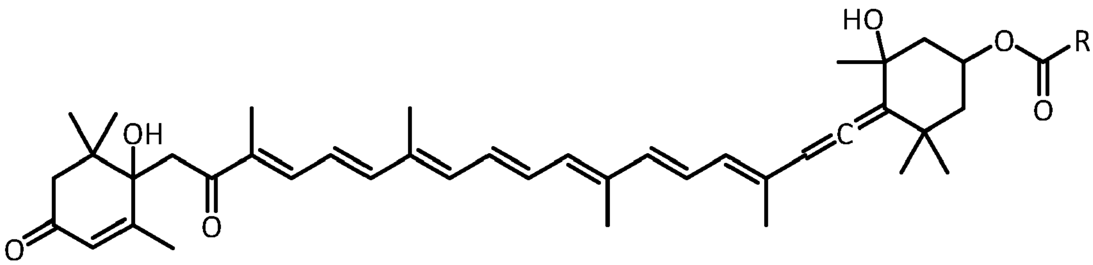

(I)

wherein R is selected from the group consisting of linear or branched $C_1$-$C_4$ alkyl, or a stereoisomer thereof and a vehicle, wherein the vehicle is selected from an oil or an emulsion.

2. The composition according to claim 1 wherein the compound has formula (Ia)

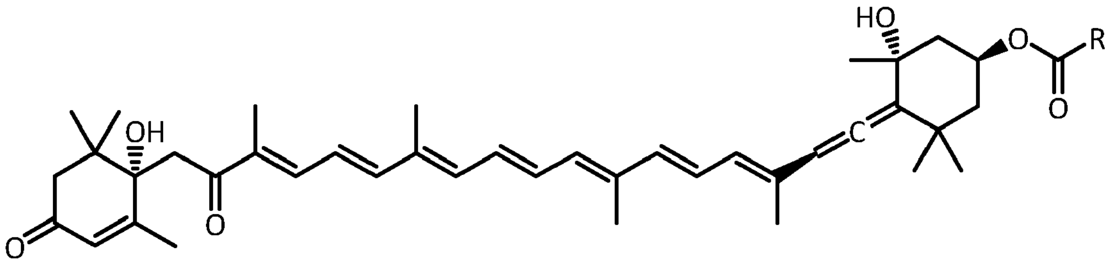

(Ia)

wherein R is as defined in claim 1.

3. The composition according to claims 1 or 2, wherein R is methyl.

4. The composition according to any of claims 1 to 3, wherein the vehicle is an oil enriched in polyunsaturated or monounsaturated fatty acids.

5. The composition according to any of claims 1 to 3, wherein the emulsion is an O/W emulsion, an O/W/O emulsion or a self-emulsifying system.

6. The composition according to any of claims 1 to 5, wherein the oil is a vegetable oil, a microorganism oil, krill oil or a fish oil.

7. The composition according to claim 6 wherein the oil is enriched in polyunsaturated fatty acids and/or omega-3 fatty acids.

8. The composition according to claim 7, wherein the vegetable oil is sunflower oil.

9. The composition according to claim 6, wherein the lipid vehicle is a fish oil or a microorganism oil which is enriched in DHA or EPA.

10. The composition according to any of the preceding claims, wherein the ratio of compound of formula (I) (expressed in milligrams) to lipid vehicle (expressed in milliliters) is from 0.01:1 to 200:1, from 0.01:1 to 150:1, from 0.01:1 to 50:1, from 0.1:1 to 50:1 or from 0.1:1 to 5:1.

11. The composition according to any of the preceding claims, wherein the concentration of the compound of formula (I) is from 0.01 to 200 mg/mL, preferably from 0.01 to 150 mg/mL, preferably from 0.01 to 50 mg/mL, preferably from 0.1 to 50 mg/mL, more preferably from 0.1 to 5 mg/mL and/or wherein the concentration of the compound according to formula (I) in the composition is from 0.01 to 200 mg/mL, preferably from 0.01 to 150 mg/mL, preferably from 0.01 to 50 mg/mL, preferably from 0.1 to 50 mg/mL, more preferably from 0.1 to 5 mg/mL.

12. The composition according to any of the preceding claims, which comprises at least 55 wt % of the compound of formula (I) with respect to the total weight of carotenoids in the composition.

13. A foodstuff, nutritional supplement, cosmeceutical, cosmetic composition or nutraceutical comprising a composition according to any of claims 1 to 7.

14. A non-therapeutic method for improving skin health comprising the step of administering to a patient in need of improved skin health a composition according to any of claims 1 to 12 or the foodstuff, nutritional supplement, cosmeceutical, cosmetic composition or nutraceutical according to claim 13.

15. The composition according to any of claims 1 to 12, or the foodstuff, nutritional supplement, cosmeceutical, cosmetic composition or nutraceutical according to claim 13 for use in the treatment and/or prevention of a disease selected form the group consisting of cancer, a cardiovascular disease, an intestinal flora imbalance, an inflammatory disease, an autoimmune disease, fibrosis, a bacterial infection, a neurological disease, a hyperuricemic-related disease, a senescence-related disorder, a lipid related disease, glucocorticoid induced muscle atrophy, a bone-related disease, an ocular disease, an angiogenic related disease, psoriasis, eczema, atopic dermatitis, a thyroid-related disorder, a disease mediated by glucocorticoid receptor activity, a senescence-associated disease or disorder, or as adjuvant in the treatment of cancer with chemotherapy, preferably taxane-comprising chemotherapy.

16. A cosmetic method for preventing and/or decreasing cutaneous senescence and/or for ameliorating the cosmetic adverse effects of aging comprising administering the composition according to any of claims 1 to 12 or the foodstuff, nutritional supplement, cosmeceutical, cosmetic composition or nutraceutical according to claim 13 to a subject in need thereof.

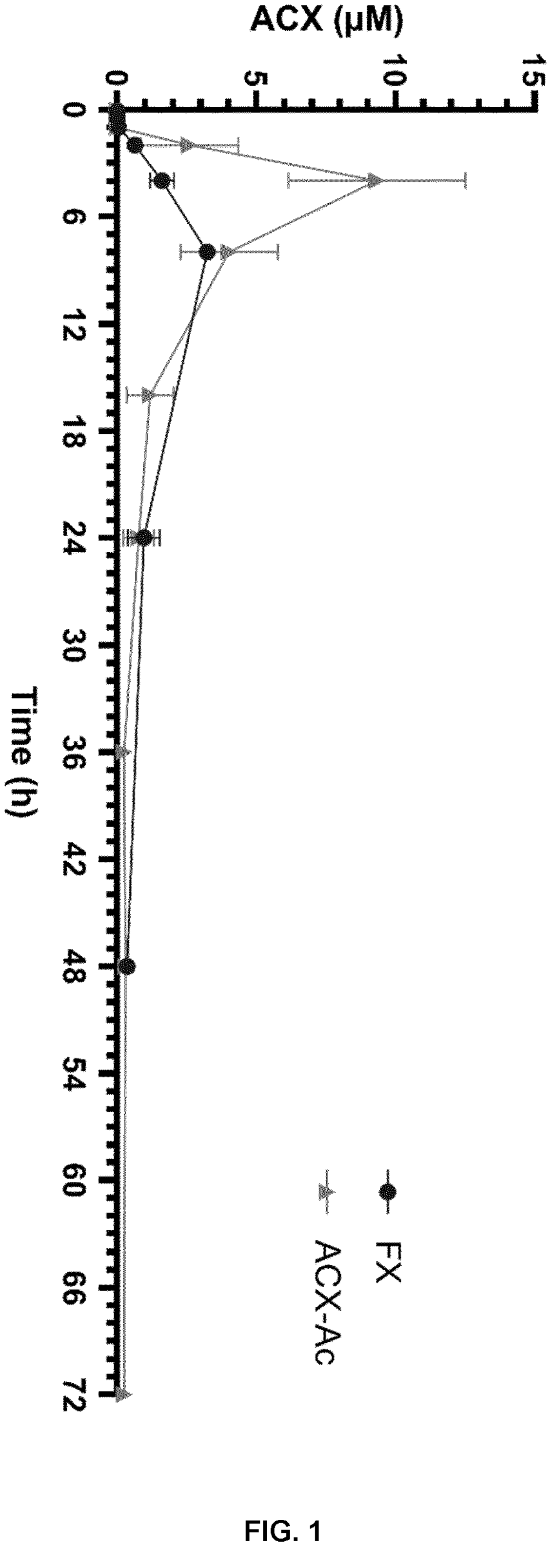


FIG. 1

Europäisches Patentamt
European Patent Office
Office européen des brevets

# EUROPEAN SEARCH REPORT

Application Number
EP 22 38 2671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAOKA TAKASHI ET AL: "New C 37 Skeletal Carotenoid from the Clam, Paphia amabillis", JOURNAL OF AGRICULTURAL AND FOOD CHEMISTRY, vol. 56, no. 24, 18 November 2008 (2008-11-18), pages 12069-12072, XP093013510, US ISSN: 0021-8561, DOI: 10.1021/jf802717b | 1-3 | INV. A61Q19/005 A61K8/49 A61K8/971 |
| Y | * abstract * * page 12070; figure 1 * * isolation of carotenoids, left column; page 12070 * ----- | 1-16 | |
| Y | WO 2015/136123 A1 (GREENALTECH S L [ES]) 17 September 2015 (2015-09-17) * page 1 * * claims 32,34,35 * * Amaroucixanthin A; page 12 * ----- | 1-16 | |
| | | | TECHNICAL FIELDS SEARCHED (IPC) |
| A | MAOKA ET AL: "Recent progress in structural studies of carotenoids in animals and plants", ARCHIVES OF BIOCHEMISTRY AND BIOPHYSICS, ACADEMIC PRESS, US, vol. 483, no. 2, 15 March 2009 (2009-03-15), pages 191-195, XP026013490, ISSN: 0003-9861, DOI: 10.1016/J.ABB.2008.10.019 [retrieved on 2008-11-01] * abstract * * page 192; compound 13 * ----- | 1-16 | A61K A61Q |

-/--

The present search report has been drawn up for all claims

1

| Place of search | Date of completion of the search | Examiner |
|---|---|---|
| Munich | 18 January 2023 | Durand-Oral, Ilknur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 38 2671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MATSUNO TAKAO ET AL: "Carotenoids of Tunicates, III. The Structural Elucidation of Two New Marine Carotenoids, Amarouciaxanthin A and B", JOURNAL OF NATURAL PRODUCTS, vol. 48, no. 4, 1 July 1985 (1985-07-01), pages 606-613, XP093013411, US ISSN: 0163-3864, DOI: 10.1021/np50040a015 Retrieved from the Internet: URL:http://dx.doi.org/10.1021/np50040a015> * the whole document * ----- | 1-16 | |
| | | | TECHNICAL FIELDS SEARCHED (IPC) |

The present search report has been drawn up for all claims

1

| Place of search | Date of completion of the search | Examiner |
|---|---|---|
| Munich | 18 January 2023 | Durand-Oral, Ilknur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 38 2671

This annex lists the patent family members relating to the patent documents cited in the above-mentioned European search report. The members are as contained in the European Patent Office EDP file on
The European Patent Office is in no way liable for these particulars which are merely given for the purpose of information.

18-01-2023

| Patent document cited in search report | Publication date | Patent family member(s) | Publication date |
|---|---|---|---|
| WO 2015136123 A1 | 17-09-2015 | EP 2918278 A1 | 16-09-2015 |
| | | EP 3116518 A1 | 18-01-2017 |
| | | WO 2015136123 A1 | 17-09-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040254357, Zaloga
- US 6245811 B, Horrobin


### Non-patent literature cited in the description


- *J Nat Prod,* 1985, vol. 48, 606-613
- *Drug. Metab. Dispos.,* 2004, vol. 32, 205-211
- *Int J Mol Sci,* 2013, vol. 14, 13763-13781
- *Biochim Biophys Acta Mol Cell Biol Lipids,* 2020, vol. 1865, 158618
- *Biochem Biophys Res Commun,* 2020, vol. 528, 305-310
- *Org Lett,* 2013, vol. 15, 5310-5313
- **ANDERSON, J. D.** *Fishery Bulletin,* vol. 105 (3), 368-378
- **SATOMI Y.** *Anticancer Res.,* 2017, vol. 37 (4), 1557-62
- **MÉRESSE S et al.** *Int J Mol Sci.,* 04 December 2020, vol. 21 (23
- **PENG J et al.** *Mar Drugs.,* 10 October 2011, vol. 9 (10), 1806-28
- **MAEDA H.** *J Oleo Sci.,* 20 January 2015, vol. 64 (2), 125-32
- **XIAO H et al.** *Mar Drugs.,* 11 December 2020, vol. 18 (12
- **SU et al.** *Front Pharmacol.,* 2019
- **LI X et al.** *Aging (Albany NY).,* 2021, vol. 13, 2655-2667
- **YANG YP et al.** *Nat Prod Res.,* June 2020, vol. 34 (12), 1791-1795
- **ZHENG J et al.** *Mar Drugs.,* 2019, vol. 17 (10), 552
- **YANG X et al.** *J Environ Pathol Toxicol Oncol.,* 2019, vol. 38 (3), 229-238
- **TAKATANI N et al.** *Biochem Biophys Res Commun.,* 23 July 2020, vol. 528 (2), 305-310
- **MA SY et al.** *Eur J Pharmacol.,* 15 September 2017, vol. 811, 199-207
- **KARPINSKI TM ; ADAMCZAK A.** Fucoxanthin-An Antibacterial Carotenoid. *Antioxidants (Basel).,* 24 July 2019, vol. 8 (8
- **HU L et al.** *Biomed Pharmacother.,* October 2018, vol. 106, 1484-9
- **WANG et al.** *Journal of King Saud University - Science,* April 2020, vol. 32 (3), 1896-1901
- **GUVATOVA Z et al.** *Mech Ageing Dev.,* July 2020, vol. 189, 111260
- **CHEN, S.-J. et al.** *Mar. Drugs,* 2021, vol. 19, 114
- **ZHIYIN L et al.** *Biomed Pharmacother.,* 14 April 2021, vol. 139, 111590
- **WALSH PJ et al.** *Mar Drugs.,* 28 February 2019, vol. 17 (3
- **CHEN S-J et al.** *Mar Drugs.,* 18 February 2021, vol. 19 (2
- **NATSUME C et al.** *Int J Mol Sci.,* 22 March 2020, vol. 21 (6
- **YANG H et al.** *Biol Trace Elem Res,* May 2021, vol. 199 (5), 1877-1884
- **KANG SY et al.** *J Cosmet Sci.,* 2020, vol. 71 (2), 53-64